(19) Europäisches Patentamt
European Patent Office
Office européen des brevets

(11) EP 1 695 724 B1

(12) EUROPEAN PATENT SPECIFICATION

(45) Date of publication and mention of the grant of the patent:
**24.01.2018 Bulletin 2018/04**

(51) Int Cl.:
***A61L 31/04*** *(2006.01)* ***A61L 31/14*** *(2006.01)*

(21) Application number: **06075900.8**

(22) Date of filing: **09.05.2001**

# (54) Fibrin material and method for producing and using the same

Fibrinmaterial und Verfahren zu seiner Herstellung und Verwendung

Matière à base de fibrine et procédé de production et d'utilisation correspondant

(84) Designated Contracting States:
**AT BE CH CY DE DK ES FI FR GB GR IE IT LI LU MC NL PT SE TR**

(43) Date of publication of application:
**30.08.2006 Bulletin 2006/35**

(62) Document number(s) of the earlier application(s) in accordance with Art. 76 EPC:
**01933275.8 / 1 387 703**

(73) Proprietor: **Baxter International Inc.**
**Deerfield, IL 60015 (US)**

(72) Inventors:
- **DELMOTTE, Yves**
  **B-7332 Neufmaison (BE)**
- **DIORIO, James**
  **Antioch, Illinois 60002 (US)**

(74) Representative: **Potter Clarkson LLP**
**The Belgrave Centre**
**Talbot Street**
**Nottingham NG1 5GG (GB)**

(56) References cited:
**WO-A-98/02098** **WO-A-98/12274**
**WO-A-99/29338** **US-A- 5 318 524**
**US-A- 5 989 215**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Technical Field

This invention provides a fibrin hydrogel material and particularly a fibrin hydrogel useful as a drug delivery vehicle and for the prevention of post surgical adhesion.

### Background Art

One of the major problems in intra-abdominal surgery is the avoidance of post-operative adhesions. It is well-known that adhesions contribute to pain, immobility, retarded wound healing, and in particular to intestinal obstruction which even may be life-threatening. In the field of gynecological surgery, post-surgical adhesions involving female reproductive organs may result in infertility.

Each surgical procedure necessarily produces various forms of trauma where the abdominal cavity or other human cavity is opened for an inspection. Physiologically, the process of wound closure then starts when bleeding ceases upon formation of a hemostatic clot at the places where blood vessels are injured. The clot, at first comprising mainly platelets, is solidified by a fibrin network resulting from the activation of an enzyme cascade involving thrombin, factor XIII and calcium. Further steps on the way to the sealing of the wound are retraction of the hemostatic clot, invasion of various cell types including fibroblasts into the wound area and eventually the lysis of the fibrin network. Adhesions are thought to begin to form when the fibrin clot covering an injury comes into contact with a bleeding adjacent surface and the new connective tissue produced by the fibroblasts attach the two surfaces together.

The problems associated with adhesions often require a further operative procedure for removing/lysing the adhesions, called adhesiolysis, which, like the first operation, principally bears the risk of forming additional adhesions.

Accordingly, the prevention of adhesion formation is medically important. Among the different approaches for prevention of adhesion formation, one involves the use of materials as aphysical orbio-mechanicalbarrierforthe separation orisolation of traumatized tissues during the healing process. Both synthetic materials and natural materials have been used as a barrier to adhesion formation. Permanent, inert implants like Gore Tex® surgical membranes consisting of expanded polytetrafluoroethylene (PTFE) generally require a second operative procedure to remove them, while others such as surgical membranes of oxidized regenerated cellulose are biodegradable, but are thought to elicit an inflammatory response ultimately leading to adhesion formation (A.F. Haney and E. Doty, Fertility and Sterility, 60, 550-558, 1993).

Fibrin sealants and glues are well-known in the art for use in haemostasis, tissue sealing, and wound healing and have been commercially available for more than a decade. Use for anti-adhesion and drug delivery vehicle in glaucoma surgical procedures is one example. Fibrin glues mimic the last step of the coagulation cascade and are usually commercialized as kits comprising two main components. The first component is a solution comprising fibrinogen with or without factor XIII, while the second component is a thrombin calcium solution. After mixing of components, the fibrinogen is proteolytically cleaved by thrombin and thus converted into fibrin monomers. Factor XIII is also cleaved by thrombin into its activated form (FXIIa). FXIIIa cross links the fibrin monomers to form a three-dimensional network commonly called "Fibrin Gel."

As disclosed in the commonly assigned published PCT patent application, WO 96/22115, a self-supporting sheet-like material of cross-linked fibrin material can be used as a bio-mechanical barrier in the treatment of internal traumatic lesions, particularly for prevention of adhesion formation as a post-operative complication. The ’115 Application discloses the mixing of a thrombin and calcium containing solution with a fibrinogen and Factor XIII containing solution. By using high thrombin concentrations to catalyze the conversion of fibrinogen into fibrin, the resulting fibrin material was found to be sufficiently rigid to be self-supporting and to have sufficiently small pore size to prevent the ingress of fibroblasts which causes the formation of adhesions. The resulting fibrin material, however, did not readily retain water. In fact water could be easily expelled from the fibrin material by compressing the material by hand. Thus, this classic type fibrin material could not be used to deliver drugs to a wound site while being reabsorbed into the body during the fibrinolytic process.

WO98/02098 discloses cross-linked fibrin material for preventing tissue adhesion. This invention overcomes these and other shortcomings in the prior art devices. Hydrogel fibrin has a tight structure constituted of thin fibers defined by a low pore size. Water is trapped in the "void volume" of the structure. The "void volume" is small, regular, and homogenously distributed through the entire film material. Water cannot leave the film structure, due to its internal energy, and is released from the fibrin structure depending on the fibrinoytic rate of the biopolymer. The release of a drug incorporated into the water or buffer is regulated by passive diffusion and, depending upon the molecular weight, solubility and the fibrinolytic process.

The removal of calcium from the process of forming a fibrin structure yields no lateral associations of protofibrils. The lack of associations of protofibrils corresponds to a high number of thin fibers per unit of volume, thus conferring a tight pore size in the fibrin structure. This tight pore size allows for water to remain trapped in the "void volume."

## Summary of the Invention

A first aspect of the invention provides a fibrin hydrogel material comprising: a fibrin material formed in the absence of a calcium containing solution or in the presence of a solution which is capable of scavenging calcium ions associated with fibrinogen molecules, which fibrin material has a water content of at least 90% by weight of the material and retains at least about 80% of the water upon compression by centrifugation at 625G for 30 minutes, and is substantially free of cross-linking.

A second aspect of the invention provides a multiple layer fibrin material comprising: a fibrin glue layer; and a layer of the fibrin hydrogel material of the first aspect of the invention.

A third aspect of the invention provides a multiple layer fibrin material comprising: a fibrin glue layer; and a layer of the fibrin hydrogel material of the first aspect of the invention, which is a therapeutic fibrin hydrogel layer.

A fourth aspect of the invention provides a multiple layer fibrin material comprising: a fibrin film layer; and a layer of the fibrin hydrogel material of the first aspect of the invention.

A fifth aspect of the invention provides the fibrin hydrogel material of the first aspect of the invention or the multiple layer fibrin material of any of the second to fourth aspects of the invention for use in medicine.

A sixth aspect of the invention provides a use of a first fibrin material in the manufacture of a medicament for treating a patient to be treated with a second fibrin material, wherein the first fibrin material has a pore size from 2 μm to 10 μm; and the second fibrin material has a pore size of from 0.1 μm to 5 μm and is capable of retaining 80-90% of water upon compression by centrifugation at 625G for 30 minutes, and is substantially free of cross-linking, and was formed in the absence of a calcium containing solution or in the presence of a solution which is capable of scavenging calcium ions associated with fibrinogen molecules; and wherein the first fibrin material is for applying to a surface and the second fibrin material is to be applied to the first fibrin material.

A seventh aspect of the invention provides a use of a second fibrin material in the manufacture of a medicament for treating a patient to be treated with a first fibrin material, wherein the first fibrin material has a pore size from 2 μm to 10 μm; and the second fibrin material has a pore size of from 0.1 μm to 5 μm and is capable of retaining 80-90% of water upon compression by centrifugation at 625G for 30 minutes, and is substantially free of cross-linking, and was formed in the absence of a calcium containing solution or in the presence of a solution which is capable of scavenging calcium ions associated with fibrinogen molecules; and wherein the first fibrin material is to be applied to a surface and the second fibrin material is for applying to the first fibrin material.

An eighth aspect of the invention provides a first fibrin material for use in treating a patient to be treated with a second fibrin material, wherein the first fibrin material has a pore size from 2 μm to 10 μm; and the second fibrin material has a pore size of from 0.1 μm to 5 μm and is capable of retaining 80-90% of water upon compression by centrifugation at 625G for 30 minutes, and is substantially free of cross-linking, and was formed in the absence of a calcium containing solution or in the presence of a solution which is capable of scavenging calcium ions associated with fibrinogen molecules; and wherein the first fibrin material is for use by applying to a surface and the second fibrin material is to be applied to the first fibrin material.

A ninth aspect of the invention provides a second fibrin material for use in treating a patient to be treated with a first fibrin material, wherein the first fibrin material has a pore size from 2 μm to 10 μm; and the second fibrin material has a pore size of from 0.1 μm to 5 μm and is capable of retaining 80-90% of water upon compression by centrifugation at 625G for 30 minutes, and is substantially free of cross-linking, and was formed in the absence of a calcium containing solution or in the presence of a solution which is capable of scavenging calcium ions associated with fibrinogen molecules; and wherein the first fibrin material is to be applied to a surface and the second fibrin material is for use by applying to the first fibrin material.

Other advantages and aspects of the present invention will become apparent upon reading the following description of the drawings and detailed description of the invention.

In a particular embodiment, the first aspect of this invention provides a medical device for the prevention of post-surgical adhesion formation and the controlled release of drugs in human and non-human species. The device comprises a fibrin hydrogel material having a water content of at least about 90% by weight of the hydrogel. The fibrin hydrogel has a pore size within the range of less than 1 micron and preferably less than 0.1 μm has a transparency of less than about 1.0 AUFS, more preferably less than about 0.8 AUFS when measured with a spectrophotometer at 800 nm. The fibrin hydrogel is substantially free of cross-linking.

In a particular embodiment, the second third or fourth aspects of the present invention further provide a multilayer fibrin material for application to animal tissue. The characteristics of each layer are determined by the concentrations of the constituents and the presence of calcium and Factor XIII. In a preferred form, in addition to a fibrin hydrogel, the material or film includes one layer of a fibrin glue. The fibrin glue layer has a pore size within the range of less than 2 to 10 microns. In another embodiment, the fibrin hydrogel includes a layer of classic fibrin film. The classic fibrin film layer has a pore size within the range of 0.1 to 10 microns and is cross-linked.

. In another embodiment, one layer of the multiple layer fibrin material is a therapeutic fibrin hydrogel material having a water content of at least 92.5 % by weight of the hydrogel and whereby the hydrogel retains 90 % of the water upon compression by a force from 1 to 14 psi. The therapeutic fibrin hydrogel layer of the multilayer fibrin material releasably retains a diluent whereby the diluent comprises a therapeutic agent. The therapeutic fibrin hydrogel layer of the multilayer fibrin material has a pore size within the range of 0.1 to 1 microns and has an optical clarity of less than about 1.0 AUFS, more preferably less than about 0.50 AUFS when measured with a spectrophotometer at 800 nm. The fibrin hydrogel layer is substantially free of cross-linking. In one embodiment of the therapeutic fibrin hydrogel layer of the multilayer fibrin material, the releasably retained therapeutic agent comprises a pharmaceutical compound. In another embodiment of the therapeutic fibrin hydrogel layer of the multilayer fibrin material, the releasably retained therapeutic agent comprises living cells, such as chondrocytes. Other cell types are contemplated as well.

## Detailed Description of the Invention

While this invention is susceptible of embodiment in many different forms, there is shown in the drawings and will herein be described in detail a preferred embodiment of the invention with the understanding that the present disclosure is to be considered as an exemplification of the principles of the invention and is not intended to limit the broad aspect of the invention to the embodiments illustrated.

One preferred form of the present invention provides, a self-supporting, biodegradable, fibrin hydrogel material which is obtained by mixing fibrinogen and thrombin solutions diluted with a solute inhibiting the action of calcium on fibrinogen in a high ionic strength medium, both free of calcium. The prior art discloses that calcium is a critical component to forming a fibrin material. The resulting hydrogel material has thin fibers and small pore size and is suitable for use as an anti-adhesion barrier.

### I. Fibrin Hydrogel Material

Preferably the fibrin hydrogel material for anti-adhesion applications will have a pore size from 1- 5 microns, and more preferably from 0.1- 3. The fibrin hydrogel material preferably also readily retains water upon compression. Preferably the hydrogel shall retain 80 - 90 % of its water content upon compressing the material with a force from 1- 14 psi.

The hydrogel material has a sufficiently high modulus of elasticity to be self-supporting. By self-supporting we mean that a fibrin hydrogel material of 5 cm long by 5 cm wide by 5mm thick can be held at one end without the second end deflecting downward with respect to the held end more than 10 degrees.

It is also desirable that the fibrin hydrogel be relatively optically transparent and, in a preferred form, should have an optical density measured with a spectrophotometer at 800 nm of from 0.1-0.2, more preferably from 0.1-0.5 and most preferably from 0.1-0.4. As can be seen in Figure 1, the fibrin hydrogel has a network of fibers 10 that should have an average diameter, in a preferred form, of less than about 5.0 microns, more preferably from less than about 2.0 microns and most preferably from less than about 1.0 microns or any range or combination of ranges therein.

In one preferred form of the invention, a fibrin hydrogel is a single layered material. The present invention further provides a multilayer fibrin material comprising two or more layers.

In preferred embodiments, the thickness of the fibrin barrier material is at least 200μm when the barrier is in the wet state. Preferably the thickness is about 50μm, and most preferably up to 10,000μun, although it is believed that even material with a thickness of less than 100μm may be suitable for the purposes of the invention.

The hydrogel material must also be capable of being reabsorbed into the body or be bioreabsorbable. Preferably, depending upon the concentration of fibrinogen and the quantity applied, a fibrin hydrogel of 3cmx3cmx1cm the hydrogel will be reabsorbed into the body in its entirety by 14 days, more preferably within 10 days and most preferably within 5 days.

The fibrin hydrogel can be distinguished from classic fibrin material in several ways. First, the fibrin hydrogel can obtain a lower pore size than that of classic fibrin material with the same concentration of thrombin. The fibrin hydrogel has a tighter pore size regardless of the concentration of thrombin used. This has an advantage over classic fibrin materials in that the fibrinolytic process is maintained or increased over physiological levels.

Classic fibrin materials utilize large quantities of calcium, thus hampering the fibrinolytic process. The fibrinolytic process is slowed by the presence of gamma-gamma cross-links in the fibrin material, which are caused by the presence of excess calcium. The lower level of calcium in the fibrin hydrogel material inhibits cross-linking, thus allowing for a faster breakdown of the fibrin hydrogel material.

The increased time required by classic fibrin materials to be broken down may also result in a greater degree of adhesion. The anti-adhesion qualities of the fibrin hydrogel are believed to result from the thrombin content. The thrombin content of the fibrin hydrogel allows for a higher fibrinolytic rate than classic fibrin materials.

Another distinction is the fibrin hydrogel's ability to retain water under compression forces. The degree of water retention in the fibrin hydrogel greatly exceeds the water retention of the classic fibrin materials. This permeability factor is a primary distinction between the fibrin hydrogel of the present invention and classic fibrin materials. The slow rate of water release by the fibrin hydrogel material allows the hydrogel to act as a lubricant by release of water, further enhancing its anti-adhesion properties.

## II. Method of Forming a Fibrin Hydrogel

It has been found by the present inventors that a fibrin hydrogel material may be formed in the absence of a calcium containing solution and in the absence of a Factor XIII containing solution which were previously considered in the art to be essential components. In a preferred form of the invention a fibrin hydrogel is obtained by admitting a fibrinogen-containing solution with a thrombin-containing solution. The fibrinogen solution should have from 1.5 - 100 mg/ml, more preferably 3 - 70 mg/ml and most preferably a 45 mg/ml fibrinogen dissolved in a solution containing components capable of chelating calcium. The chelating component should also be non-toxic and in a preferred form of the invention is a phosphate buffer saline solution (PBS) of physiologically acceptable levels. The chelating agent should be an antagonist to fibrinopeptide transmidation reaction 5 IU-300 IU.

Also, contrary to the teachings in the prior art, the thrombin concentration of the admixed components does not determine the pore size of the hydrogel fibrin material. As will be discussed below and as shown in Figures 1-5, fibrin hydrogel materials were formed having relatively the same pore size notwithstanding the use of thrombin concentrations from 1 IU to 300 IU. The concentration of thrombin was still found to control the rate of forming a fibrin hydrogel.

It is well known that mixing a first solution containing fibrinogen with Factor XIII and a second solution of thrombin with calcium will result in the formation of a fibrin material with pronounced lateral association and considerable cross-linking among its thick fibers. It is also well known that thrombin acts as a protease which will cleave fibrinopeptide A and B from the fibrinogen molecule and convert it into fibrin. The fibrinopeptides of vertebrate species reportedly have a large net negative charge. The presence of these and other negatively charged groups in the fibrinopeptides are likely actors in keeping fibrinogen apart. Their release by thrombin gives fibrin monomers a different surface-charge pattern, leading to their specific aggregation. In particular, removal of the fibrinopeptides changes the net charge of the central globular unit from -8 to +5. Each of the terminal globular units has a net charge of -4. Thus, electrostatic interactions between the terminal and central globular units probably stabilize the structure of fibrin.

It is also known that calcium ions play an important role in the dissociation of Factor XIII subunit A from Factor XIII subunit B as Factor XIII is converted to its activated form, Factor XIIIa. Furthermore, it is know that Factor XIIIa is critical to the cross-linking of fibrin monomers. It is also known that the widths of the fibers comprising the fibrin material can be decreased by increasing the pH and ionic strength of the diluents.

By removing (chelating) calcium ions bound to fibrinogen, the inventors have been able to modify the fibrin structure to obtain further embodiments of the fibrin hydrogel. Accordingly, the present invention uses a solution capable of scavenging calcium ions associated with the fibrinogen molecules. In a preferred form of the invention, a phosphate buffer solution having a concentration similar to physiologically acceptable levels. The inventors suggest that the resulting structural modifications of the fibrin hydrogel occur as a result of a "charge effect" which alters the aforementioned electrostatic interactions between the terminal and central globular units, thereby inhibiting the lateral association of fibrin. In further embodiments of the fibrin hydrogel, modification of Factor XIII concentration used in the synthesis of fibrin alters the crosslinking characteristics of the final fibrin material. Thus, in further embodiments of the invention, the inventors have developed a fibrin hydrogel that can be synthesized with low concentrations of thrombin and according to the end user’s specifications as to the lateral association and fiber thickness of the resulting fibrin hydrogel structure.

During the formation of a fibrin hydrogel, it is desirable that all of the fibrinogen be converted into fibrin, as residual amounts of fibrinogen may lead to adhesion formation upon reacting with thrombin present in the body. Accordingly, in still further embodiments of the present invention, the fibrin hydrogel further comprises less than 5 % by weight of fibrinogen, preferably less than 4% by weight of fibrinogen, preferably less than 3% by weight of fibrinogen, preferably less than 2% by weight of fibrinogen, and most preferably less than 1% by weight of fibrinogen, in terms of the total dry weight of the fibrinogen plus fibrin each time. For the purpose of determining the fibrin and the fibrinogen content of the fibrin film, the methods of SDS-Page (SDS-Gel Electro-phoresis) may be used.

The medical devices shown in Figure 6 and as further described in commonly assigned U.S. Patent No. 5,989,215 may be used topically, in open-type surgeries (for example, laparotomic surgeries) or minimally invasive surgeries (for example, laparoscopic surgeries). Of course, there are other types of open-type surgeries and minimally invasive surgeries as will be appreciated by one of ordinary skill in the art. The medical device 20 may be used to form fibrin hydrogel material inside and outside the animal body.

The present invention provides a process for preparing a self-supporting fibrin hydrogel matrix or film outside the body comprising the steps of:

(a) mixing a stream of a first, fibrinogen-containing solution dissolved in PBS with a stream of a second, thrombin-containing PBS solution;
(b) applying the obtained mixture to a solid support or mixing the components of the solid support; and
(c) incubating the mixture to form the hydrogel matrix.

In order to obtain a mixture as homogenous as possible (and thus a homogenous final product) in step (a) a stream of a first, fibrinogen-containing solution is mixed with a stream of a second, thrombin-containing solution by simultaneous delivery of the components. It is also possible to deliver one component to a surface followed by the other component. Preferably, equal volumes of the first and the second solution are mixed. In case the different volumes of the first and the second solution should be mixed, it will be known in the art which measures have to be taken in order to ensure that a homogenous mixture is obtained.

Using the delivery device mentioned above, the resulting mixture is spread over the surface of a solid support, for example a petri dish or the like, which is tilted to cover the entire surface as far as possible before the formation of the fibrin hydrogel material begins.

For the purpose of preparing a fibrin hydrogel on mammal tissue, the inventors propose a process comprising the steps of:

(a) providing a first phosphate buffer solution containing fibrinogen;
(b) providing a second phosphate buffer solution containing thrombin;
(c) mixing the first solution and the second solution before or after placing the mixture on an animal tissue;
(d) and obtaining a fibrin hydrogel material with a tight structure and small pore size suitable for post-surgical adhesion prevention.

The fibrinogen and thrombin solutions can be initially mixed in a delivery device, or be atomized into a spray and mixed while in the form of spray droplets while in mid air or upon first making contact with the tissue surface or delivered through a multi-lumen catheter.

The fibrin hydrogel may be formed by utilizing constituents of a kit. A preferred embodiment of the fibrin hydrogel kit includes:

(a) a vial of proteins including fibrinogen;
(b) a vial of thrombin;
(c) a vial of phosphate buffer solution to serve as a diluent; and
(d) appropriate ancillary mixing and application apparatus, including, but not limited to syringes and catheters.

One further embodiment of the fibrin hydrogel kit includes a vial containing the protein cocktail where the protein content is no less than 30 mg/ml. Another further embodiment of the fibrin hydrogel kit includes a vial containing the protein cocktail where the Factor XIII content ranges from 0 IU/ml to 80 IU/ml. Yet a further embodiment of the fibrin hydrogel kit includes a vial containing thrombin, where the concentration of thrombin ranges between 0.1 IU/ml to 1000 IU/ml. In yet another preferred embodiment of the fibrin hydrogel kit, the constituents supplied are pre-formulated to ensure that when mixed, the hydrogel achieved will have a homogeneous structure with tight pore sizes suitable to act as a prophylaxis to adhesion formation.

Another preferred embodiment of the fibrin hydrogel kit includes pre-formulated constituents, supplied to ensure that when mixed, the hydrogel achieved will have a homogeneous structure with tight pore sizes suitable to act as a prophylaxis to adhesion formation and as a drug delivery system. A lack of adhesion can be seen with in fibrinogen free of FXIII or similar acting component. The fibrin hydrogel kit includes a vial of fibrinogen mixed with inactivated thrombin, a vial of suitable buffer, an ancillary, a device equipped with optical fiber to photoactivate the thrombin, and a light supply. In another preferred embodiment of the fibrin hydrogel kit, the fibrinogen, inactivated thrombin, suitable buffer, and an ancillary are all in one delivery device. Figure 7 illustrates another preferred embodiment where a pressurized canister houses fibrinogen and thrombin as powder in separate bags. The canister may be unscrewed to allow for rehydration of the fibrinogen and thrombin. Increasing the pressure allows both components to be sprayed through a double tube system or through concentric channels. Another preferred embodiment, Figure 8, utilizes a double syringe system with volumes of less than 100, 50, 20 ml each, more preferably less than 20 ml each, and most preferably less than 10 ml each, but greater than 3.0 ml. This double syringe system is equipped with a Y-shaped connection to incorporate a tube. Such devices can be used for veterinary applications. In another preferred embodiment, the fibrin hydrogel material can be fabricated into articles selected from the group consisting of films, tubes, and pellets. These fibrin hydrogel materials can be fabricated into articles using techniques selected from the group of extrusion, molding, and thermal forming. These fibrin hydrogel materials can be sterilized at a temperature below 0°C by gamma radiation, stored at a temperature below 0°C, and used upon demand. The sterilization by gamma radiation is below - 25 °C, at a dosage of at least 25 kGy. In yet another preferred embodiment of the fibrin hydrogel kit, the vial of diluent contains a phosphate buffer solution. In another preferred embodiment of the fibrin hydrogel kit, the vial of diluent contains a high-ionic strength buffer capable of scavenging the fibrinogen-linked calcium. Other suitable solutions include; sodium citrate, potassium citrate, EDTA, EGTA, chloride solutions, phosphate solutions, or other ions solutions having a strong affinity for calcium. Fibrinogen, thrombin, and other proteins such as fibronectin and FXIII may be from a single donor, multiple donors, pooled donors, Cohn I fraction, or recombinant. In another preferred embodiment of the fibrin hydrogel kit, the vial of diluent contains a buffer capable of chelating exogenous calcium.

**III. Therapeutic Fibrin Hydrogel**

The present invention further provides a fibrin hydrogel that releasably retains a diluent or a therapeutic agent. The therapeutic agent is retained within the pores of the hydrogel material and when placed into the body of a mammal is released over time as the fibrin hydrogel is reabsorbed into the body.

The therapeutic agent(s) that are contemplated to be releaseably retained by the therapeutic hydrogel layer comprises, but is not limited to, pharmaceutical compounds, antibiotics, fibrinolytic agents, and biological response modifiers, inparticular cytokines and wound repair promoters, preferably in an amount up to 1 % by weight in terms of the total dry weight of fibrin plus fibrinogen. Due to the chemotactive properties of thrombin, low thrombin concentration is preferred for the purpose of anti-adhesion,. However, higher concentrations of thrombin may be required to hasten clotting time. Clotting time was performed with a semi-automated BFT II device from Dade Behring on fibrinogen at 25 mg/ml with varying thrombin concentrations of 0.5, 1.0, 2.5, 5.0, and 10.0 IU/ml. PBS was used as a diluent for fibrinogen and thrombin. The clotting times are listed in the table below.

| Fibrinogen Concentration | Thrombin Concentration | Clotting Time (Seconds) |
|---|---|---|
| 25 mg/ml | 0.5 IU/ml | 430 |
| 25 mg/ml | 1.0 IU/ml | 237 |
| 25 mg/ml | 2.5 IU/ml | 83 |
| 25 mg/ml | 5.0 IU/ml | 37 |
| 25 mg/ml | 10.0 IU/ml | 20 |

Examples of fibrinolytic agents include t-PA, μ-PA, streptokinase, staphylokinase, plasminogen and the like. These compounds promote fibrinolysis and thus can be used for controlling the rate of the degradation of the fibrin film in vivo. The term "*biological response modifiers*" is meant to refer to substances which are involved in modifying a biological response, such as wound repair, in a manner which enhances the desired therapeutic effect. Examples include cytokines, growth factors, and the like. Due to its intrinsic mechanical properties, the fibrin film of the invention does not require any additional cross-linking agent which may exert any toxical effects to the human or animal body. Due to its high level of dilution, it is possible for the fibrin hydrogel to trap and release water. This is useful for the hydration of tissues or as a lubricant to assist in the anti-adhesive properties of the fibrin hydrogel.

The therapeutic agent can be incorporated into the fibrin hydrogel material during the formation of the hydrogel. The therapeutic agent may either water soluble or water insoluble, antibody, antimicrobial agent, agent for improving biocompatability, proteins, antiinflammatory compounds, compounds reducing graft rejection, living cells, cell growth inhibitors, agent stimulating endothelial cells, antibiotics, antiseptics, analgesics, antineoplastics, polypeptides, protease inhibitors, vitamins, cytokines, cytotoxins, minerals, interferons, hormones, polysacharides, genetic material, growth factors, cell growth factors, substances against cholesterol, pain killers, collagens, stromal cells, osteo-progenitor cells, polylactate, alginate, $C_2$-$C_{24}$ fatty acids, and mixtures thereof. The delivery of the therapeutic agent regulated by either or both the passive diffusion and the fibrinolytic rate. The therapeutic agent can be dissolved in one or both of the thrombin or fibrinogen solutions. The therapeutic agent is retained by the hydrogel material as it forms out of the admixed solution.

**IV. Multilayer Fibrin Hydrogel Film**

The present invention further provides a multiple layer fibrin hydrogel film. The fibrin hydrogel can include a single additional layer or multiple additional layers of fibrin glue, classic fibrin film, fibrin hydrogel, therapeutic fibrin hydrogel film and layers of other synthetic or naturally occurring materials, such as alginate, polylactic, glycolic, silicon, and hyluronic compounds. The present invention contemplates this material being bound to the surface of synthetic polymers by modifying the surface biomechanically or otherwise altering the physical retention of the surface. Additionally, partiallypremixing the components at the interface between the layers may allow for bonding to occur. Furthermore, binding to collagen or other organic material is also contemplated. Collagen and other organic materials have a chemical affinity for proteins such as fibrinogen and fibronectin. The present invention contemplates selecting any combination of the above components and connecting them together in differing orders based upon the desired function of the film material. The present invention further contemplates selecting the individual thicknesses of the individual layers and the overall thickness of the film based upon its intended function. The following is a set of non-limiting examples of multiple layered films. The present invention should not be limited to these exemplary embodiments.

The present invention provides a multiple layered fibrin film having a first layer of a fibrin hydrogel or therapeutic hydrogel and a second layer of a classic fibrin film. The "classic" fibrin film is obtained by mixing a thrombin and calcium containing solution with a fibrinogen and Factor XIII containing solution as disclosed in detail in PCT Application WO 96/22115. The first and second layers readily adhere to one another. During the conversion process, the adhesive property of fibrinogen is present and can allow the layers to stick together. If the fibrinogen is added too late, the obtained fibrin material is no longer adhesive, thus resulting in the possibility of delamination. It is contemplated by the present invention that mechanical retention can be inhanced by making holes in the first layer where the fibrin glue can penetrate and adhere the layers.

The present invention also provides a three-layered film having a first layer of a fibrin hydrogel, an inner layer of fibrin glue and an outer layer of a therapeutic hydrogel material. Another three-layered film includes inner and outer layers of fibrin glue on opposed surfaces of a layer of fibrin hydrogel material. Preferably the fibrin glue is obtained by mixing of fibrinogen-containing solution with an equal volume of a thrombin-containing solution. The fibrinogen-containing solution contains fibrinogen and factor XIII (0.1 - 40 IU/ml). The concentration of fibrinogen is expressed as the total protein concentration (preferably from about 3 -140 mg/l and more preferably 30-110mg/ml) and the percentage of clottable protein therein.

It is also preferred that the fibrinogen solution have a viscosity that allows the solution to be sprayed and preferably sprayed using pressures generated using a hand-operated syringe. The fibrinogen solution should have a viscosity of less than 20 centipoise, more preferably less than 10 centipoise, and most preferably from 1-5 centipoise or any combination or subcombination of ranges therein. The thrombin-containing solution should have a thrombin concentration less than 10000 IU thrombin. The fibrin glue has been preferably made by mixing said fibrinogen-containing solution with an equal volume of a thrombin-containing solution of at least 50 IU thrombin, preferably of at least 150 IU thrombin, and most preferably of at least 300 IU thrombin

Yet another example of a multilayered film includes layers stacked in the order of classic fibrin film/hydrogel/therapeutic hydrogel/hydrogel/classic fibrin film. In this case the delivery of the therapeutic agent in the therapeutic hydrogel can be delayed by the time it takes for the outer layers to be reabsorbed into the body. Thus, the present invention provides for building into the structure of the multilayered film time delivery sequences or schemes as desired.

Other contemplated embodiments include:

1. A multilayered structure composed of a surface layer of hydrogel material and a bottom layer of membrane. The membrane may be tissue or fibrin.
2. A multilayered structure composed of a surface layer classic fibrin and a bottom layer of hydrogel material.
3. A multilayered structure composed of outer layers of classic fibrin and an inner layer of hydrogel material.
4. A multilayered structure composed of a surface layer of hydrogel material and an inner layer of fibrin sponge material.
5. A multilayered structure composed of outerlayers of hydrogel material and an inner layer of membrane.
6. Beads of hydrogel material between 0.1mm and 3mm.
7. A hydrogel material anatomically molded.

The present invention also provides a process for preparing a multilayer fibrin material. One such process for preparing a multilayer fibrin material includes the steps of:

(1) providing a base fibrin hydrogel layer, comprising the steps of:

(a) providing a first buffer solution containing fibrinogen;
(b) providing a second buffer solution containing thrombin;
(c) providing additional constituents in either the first or second buffer solutions as required for a specific preparation;
(c) mixing the first solution, the second solution, and any additional solutions on a surface such as a petri dish or tissue;
(d) and obtaining a fibrin layer with a desired structure and desired pore size suitable for its designated purpose.

(2) providing an additional layer by repeating steps 1(a) through 1(d) wherein the mixing occurs on the earlier formed layer or layers;
(3) providing additional layers, if desired, by repeating step 2;
(4) providing a final layer by repeating steps 1 (a) through 1 (d).

The present invention further provides a fibrin hydrogel that retains a higher proportion of water than fibrin materials currently available. The greater degree of water retention is particularly beneficial to the therapeutic use of the hydrogel. The retention of water is necessary for the control of the concentration of therapeutic agents contained within the fibrin hydrogel, as well as for the effective release of these therapeutic agents and additives.

The ability of fibrin hydrogels to retain water while being subjected to compression forces was tested and compared to the water retaining capacity of a classic fibrin material. In particular, compressionwas applied by centrifugation of the materials at various rotational speeds and the amount of water retained was measured. A refrigerated centrifuge (Sorvall RT 6000B) spun fibrin hydrogels at different speeds:

- 1000 rpms for 30 min. corresponding to 156G
- 2000 rpms for 30 min. corresponding to 625G
- 3000 rpms for 30 min. corresponding to 1428G

Amicon filter type "centricon 30" was used, corresponding to a membrane cutoff of 30000 and characterized by a maximum rotation time of 30 min and sustaining a G-force max of 5000G. The Amicron filter is composed of two units. The upper unit contains the filter component itself and can be attached to the second unit of the Amicron filter. The second, or lower, unit is the bottom cup. The bottom cup allows for the collection of water that is expelled from the fibrin material deposited on the filter of the upper unit. The water collected in the bottom cup is used to measure the amount of water released by the fibrin materials at the various rotational speeds. Once fibrinogen and thrombin solutions were prepared, a volume of approximately 1 ml of fibrin was applied to the filter. An appropriate mixing device is required for the fibrinogen and thrombin mixture to be complete and homogeneous.

Upper and lower parts are separately weighed before the fibrin material deposition and after each centrifugation step. A correction factor is calculated in order to consider that 1 g of fibrin has been distributed on the filter.

Separate experiments were conducted to test the effects of diluents. The procedural steps for each experiment went as follows:

1) The filter and the bottom cup of the Amicon filter are separately weighed, then the fibrin material obtained by mixing each fibrinogen solution with a 20IU/mL, thrombin solution is put on the filter.
A correction factor is calculated in order to consider that 1 g of fibrin has been distributed on the filter.
The filter is centrifuged at 1000 rpm for 30 minutes.
At the end of the centrifugation cycle, the bottom cup is carefully removed, weighed and recorded.
2) The bottom cup is connected to the filter and centrifuged at 2000 rpm for 30 minutes, at the end of the cycle, the bottom cup is weighed and the cumulative value recorded.
3) Again the bottom cup is connected to the filter and centrifuged at 3000 rpm for 30 minutes and the bottom cup weighed at the end of the cycle.

In the first experiment, the fibrinogen vial was reconstituted with 3.5 ml- distilled water to obtain a final concentration of 100 mg/ml of fibrinogen. Dilutions of the fibrinogen were performed with water in order to respectively obtain:

- dilution 1:2 (50mg/ml) in water
- dilution 1:4 (25mg/ml) in water
- dilution 1:6 (16.6mg/ml) in water
- dilution 1:8 (12.5mg/ml) in water

Thrombin (Baxter Hyland) was reconstituted with 3.5 ml of 40mmol $CaCl_2$ in order to obtain a concentration of 300IU/mL. A dilution is performed with $CaCl_2$ to obtain a thrombin concentration of 20IU/mL.

Fibrinogen solutions were then mixed with an equal volume of thrombin (20IU/mL) to obtain a final concentration of "fibrinogen" respectively of:

- Sample 1: diluted 1:4 (25mg/ml)
- Sample 2: diluted 1:8 (12.5mg/ml)
- Sample 3: diluted 1:12 (8.3mg/ml)
- Sample 4: diluted 1:16 (6.25mg/ml)

For sample 1, the loss of water was 9.5%, 25%, and 45% at 1000, 2000, and 3000 rpm respectively. Sample 2 showed a loss of water of 18%, 40%, and 70% at 1000, 2000, and 3000 rpm respectively. The loss of water in sample 3 was 20%, 40%, and 80% at 1000, 2000, and 3000 rpm respectively. Sample 4 expressed a water loss of 20%, 72%, and 90% at 1000, 2000, and 3000 rpm respectively. See Table 1 below.

Table 1
Fibrinogen & Thrombin in Water

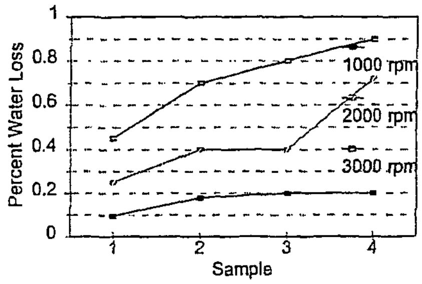


The second experiment was a comparison of water retention between fibrin obtained by mixing fibrinogen and thrombin respectively reconstituted and diluted in PBS and fibrinogen reconstituted and diluted in PBS with thrombin reconstituted and diluted in 40mmol calcium chloride.

In these comparisons, a vial of fibrinogen was reconstituted with 3.5 ml PBS (phosphate buffered saline pH=7.2) to reach a final concentration of 100mg/mL of fibrinogen.

Dilutions were performed from this vial in order to obtain fibrinogen concentrations of:

- Sample 11:2 (50mg/ml) in PBS
- Sample 2 1:4 (25mg/ml) in PBS
- Sample 3 1:6 (16.6mg/ml) in PBS
- Sample 4 1:8 (12.5mg/ml) in PBS

In experiment 2 A, thrombin (Baxter Hyland) was reconstituted with 3.5 mL of PBS in order to obtain a concentration of 300IU/mL. A dilution was performed with PB S to obtain a thrombin concentration of 20IU/mL.

In experiment B, thrombin (Baxter Hyland) was reconstituted with 3.5 ml of 40mmol $CaCl_2$ (calcium chloride) in order to obtain a concentration of 300IU/mL. A dilution was performed with cacl2 to obtain a thrombin concentration of 20IU/mL.

The fibrinogen solutions were then mixed with an equal volume of thrombin (20IU/mL), resulting in final concentrations for fibrinogen of:

- Sample 1: diluted 1:4 (25mg/ml) in PBS
- Sample 2: diluted 1:8 (12.5mg/ml) in PBS
- Sample 3: diluted 1:12 (8.3mg/ml) in PBS
- Sample 4: diluted 1:16 (6.25mg/ml) in PBS

For experiment 2 A, sample 1 expressed a loss of water that was 6%, 10%, and 14% at 1000,2000, and 3000 rpm respectively. Sample 2 showed a loss of water of 10%, 21%, and 35% at 1000, 2000, and 3000 rpm respectively. The loss of water in samples 3 and 4 was nearly identical at 11%, 20%, and 35% at 1000,2000, and 3000 rpm respectively. See Table 2A below.

Table 2A

PBS Diluent for Fibrinogen & Thrombin

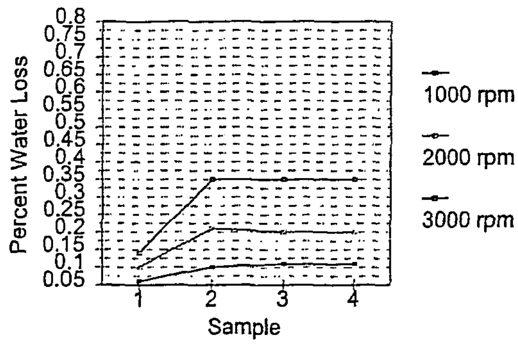


The introduction of calcium to the fibrin formulation through the diluent used for the thrombin dilutions in experiment 2 B directly affected the water retention. The loss of water was not significant at 1000 rpm for the samples, but water losses increased significantly to approximately 40% at 2000 rpm for samples 2 through 4. At 3000 rpm, water loss increased to approximately 65% for samples 2 and 3. A water loss of 80% for sample 4, similar to the result obtained for fibrin described in experiment 1, was recorded at 3000 rpm. The results for these experiments support the hypothesis that fibrin structures essentially free of calcium ions are also tighter, more compact, and have a greater resistance to water loss from compression forces. See Table 2B below.

Table 2B: PBS Diluent for Fibrinogen CaCl2 for Thrombin (20IU)

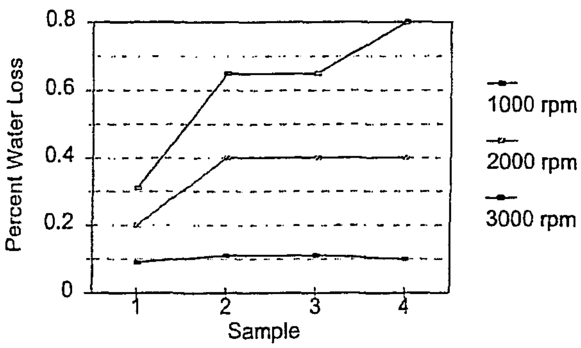


As a means of verification for the role of phosphate as a complexing agent of the remaining calcium ions on fibrinogen molecules, reproductions of the PBS experiment above were conducted substituting EDTA for PBS in one trial, and citrate of potassium for PBS in a second trial. The patterns of water loss for both the EDTA and citrate of potassium trials were consistent with the results from the experiment utilizing PBS as a reconstitution agent. See Tables 3A and 3B below. These results sustain the hypothesis that remaining calcium on fibrinogen reacts with phosphate ions preventing the collateral association of protofibrils producing a tight fibrin structure more resistant to water loss than fibrin structures retaining calcium.

Table 3A
Fibrinogen (EDTA 50 mM)

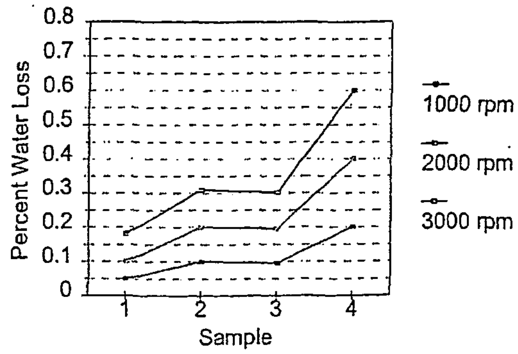


Table 3B: K Citrate as Diluent for Fibrinogen and Thrombin

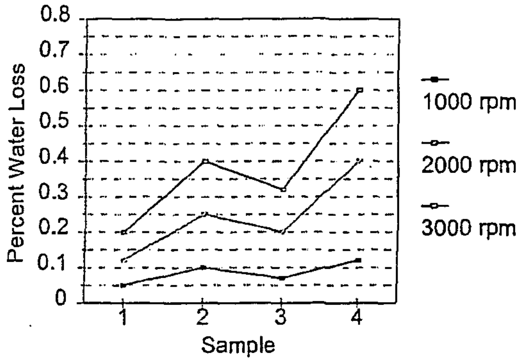


Another experiment was conducted to determine the impact of the FXIII present in the formulation on the compaction capability of the fibrin material obtained with PBS as diluent for both fibrinogen and thrombin. In this experiment, a vial of fibrinogen (Tisseel from Baxter Hyland-Immuno lot P5488797D) is reconstituted with 4.0 ml of PBS (dilution 1:2) to reach a final concentration of 50mg/mL of fibrinogen.

Dilutions are performed from this vial in order to obtain a fibrinogen concentration respectively of:

- Dilution 1:2 (50mg/ml) in PBS
- Dilution 1:4 (25mg/ml) in PBS
- Dilution 1:6 (16.6mg/ml) in PBS
- Dilution 1:8 (12.5mg/ml) in PBS

Thrombin (Baxter Hyland) is reconstituted with 3.5 ml of PBS in order to obtain a concentration of 300IU/mL. A dilution is performed with PBS to obtain a thrombin concentration of 20IU/mL. The results of this experiment show that there is no difference between the Baxter Fibrin sealant containing FXIII (experiment 2) and the Baxter Hyland-Immuno free of FXIII when submitted to the compaction test. Results obtained from the compaction tests show the same behavior for the FXIII free sealant. See Table 4 below.

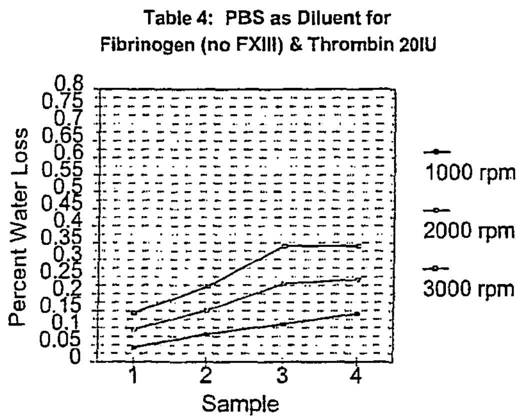


A table summarizing the water retention data follows below.

Table 5

| Sample # | Experiment # | % Water loss at 1000 rpm | % Water loss at 2000 rpm | % Water loss at 3000 rpm |
|---|---|---|---|---|
| 1 | 1 | 9.5 | 25 | 45 |
| 1 | 2A | 6 | 10 | 14 |
| 1 | 2B | not significant | 40 | 65 |
| 1 | 4 | 4.2 | 9.4 | 14.3 |
| 2 | 1 | 18 | 40 | 70 |
| 2 | 2A | 10 | 21 | 35 |
| 2 | 2B | not significant | 40 | 65 |
| 2 | 4 | not significant | 15 | 22 |
| 3 | 1 | 20 | 40 | 80 |
| 3 | 2A | 11 | 20 | 35 |
| 3 | 2B | not significant | 40 | 65 |
| 3 | 4 | not significant | 23 | 34 |
| 4 | 1 | 20 | 72 | 90 |
| 4 | 2A | 11 | 20 | 35 |
| 4 | 2B | not significant | - | 80 |
| 4 | 4 | 14 | 24 | 34 |

It has been postulated that ionic strength of thrombin regulates the pore size of fibrin clot structure. By using high ionic strength thrombin solutions one can achieve a fibrin clot having a smaller pore size than with lower concentration thrombin solutions. The present example demonstrates a method for fabricating a fibrin clot material where the concentration does not govern the pore size of the fibrin clot. As set forth above, by using a chelating agent to bind to calcium, calcium concentration does not affect the pore size. Even when a 4 IU/ml thrombin concentration and a 250 IU/ml thrombin concentration was used, the resulting fibrin material had substantially the same pore size. Ionic strength was measured with an osmometer and correlated with the measurement of the turbidity at 800 nm with a spectrophotometer. Observation of the sample network structure was accomplished by scanning electron microscopy. Table 6 below summarizes the correlation between final osmolarity of the fibrin samples and their respective optical densities. The results of this experiment illustrate that ionic strength, as demonstrated through osmolarity, does not regulate the structure of the fibrin clot.

Classic fibrin materials obtained by using water (0 mosm) as a diluent for fibrinogen and $CaCl_2$ for thrombin (4 IU/ml), for example, has a final osmolarity of 539 mosm. This is a result of fibrinogen reconstituted with water at a concentration of 90 mg/ml (610 mosm) combined with thrombin reconstituted with $CaCl_2$ at a kit concentration of 4 IU/ml (468 mosm). The resulting classic fibrin material is opaque white with an optical density of 2.8 AUFS when measured with a spectrophotometer at 800 nm.

Fibrin materials produced with PBS (286 mosm) have a final osmolarity of 445 mosm. This is a result of fibrinogen reconstituted and diluted with PBS at a concentration of 25 mg/ml (588 mosm) combined with thrombin reconstituted and diluted with PBS at a concentration of 10 IU/ml (315 mosm). The resulting fibrin material is optically clear with an optical density of 0.5 AUFS when measured with a spectrophotometer at 800 nm.

Fibrin materials produced with a citrate buffer at 0.033 M (100 mosm) have a final osmolarity of 224 mosm. This is a result of fibrinogen reconstituted and diluted with citrate at a concentration of 50 mg/ml (336 mosm) combined with thrombin reconstituted and diluted with citrate at a concentration of 20 IU/ml (112 mosm). The resulting fibrin material is optically clear with an optical density of 0.45 AUFS when measured with a spectrophotometer at 800 nm. The fibrin hydogel material in this experiment is clear and composed of thin fibers with an ionic strength of less than 300 mosm, which is considered to be the physiological level. Fibrin obtained by mixing fibrinogen at 12.5 mg/ml with thrombin at 10 IU remains clear (0.8 AUFS) with an osmolarity of 167 mosm.

Fibrin materials produced with a citrate buffer at 0.066 M (190 mosm) have a final osmolarity, of 317 mosm. This is a result of fibrinogen reconstituted and diluted with citrate at a concentration of 50 mg/ml (435 mosm) combined with thrombin reconstituted and diluted with citrate at a concentration of 20 IU/ml (200 mosm). The resulting fibrin material is optically clear with an optical density of 0.23 AUFS when measured with a spectrophotometer at 800 nm. The fibrin hydogel material in this experiment is also clear and composed of thin fibers with an ionic strength of less than 300 mosm, which is considered to be the physiological level. Fibrin obtained by mixing fibrinogen at 12.5 mg/ml with thrombin at 10 IU remains clear (0.5 AUFS) with an osmolarity of 260 mosm.

Table 6

| Buffer | Final Osmolarity of Fibrin Material (mosm) | Optical Density (AUFS at 800 nm) |
|---|---|---|
| Water ($CaCl_2$) | 539 | 2.8 |
| PBS | 451 | 0.255 |
| Citrate (0.033 M) | 224 | 0.45 |
| Citrate (0.066 M) | 317 | 0.23 |

The buffers also play an active role in the permeability, fiber diameter, and mass length ratio of the fibrin material. Significant differences can be observed between PBS and NaCl (0.15 M). These buffers have the same osmolarity, yet their effects on fibrin materials are markedly different. See Table 7 below. For a thrombin concentration of 2IU/ml reconstituted with NaCl at 0.15 M, the permeability of the fibrin is 30.6 x $10^{-12}$ at a fibrinogen concentration of 25 mg/ml and 136 x $10^{-12}$ at a fibrinogen concentration of 12.5 mg/ml. Using PBS as a buffer yields a fibrin permeability of 6.9 x $10^{-12}$ at a fibrinogen concentration of 25 mg/ml and 39.5 x $10^{-12}$ at a fibrinogen concentration of 12.5 mg/ml. This experiment demonstrates that the fibrin material utilizing PBS as a buffer is nearly five times less permeable than classic fibrin materials, thus capable of retaining more water. The diameters of fibers are also affected by the buffer selected. For a thrombin concentration of 2 IU/ml diluted with NaCl at 0.15 M, the fibers have a diameter of 0.107 $\mu$m at a fibrinogen concentration of 25 mg/ml and 0.14 $\mu$m at a fibrinogen concentration of 12.5 mg/ml. Using PBS as a buffer yields fibers with a diameter of 0.051 $\mu$m at a fibrinogen concentration of 25 mg/ml and 0.075 $\mu$m at a fibrinogen concentration of 12.5 mg/ml. Additionally, the mass length ratio is also affected by the buffer selected to reconstitute the fibrinogen and thrombin. At a thrombin concentration of 2 IU/ml buffered with NaCl at 0.15 M, the mass length ratio is 8.1 x $10^{12}$ at a fibrinogen concentration of 25 mg/ml and 13.9 x $10^{12}$ at a fibrinogen concentration of 12.5 mg/ml. The use of PBS as a buffer yields fibers with a mass length ratio of 1.83 x $10^{12}$ at a fibrinogen concentration of 25 mg/ml and 4.03 x $10^{12}$ at a fibrinogen concentration of 12.5 mg/ml, a four-fold reduction under NaCl at 0.15 M.

For a thrombin concentration of 250 IU/ml (Table 8) diluted with NaCl at 0.15 M, the permeability of the fibrin is 14.24 x $10^{-12}$ at a fibrinogen concentration of 25 mg/ml and 47.7 x $10^{-12}$ at a fibrinogen concentration of 12.5 mg/ml. Using PBS as a buffer yields a fibrin permeability of 8.9 x $10^{-12}$ at a fibrinogen concentration of 25 mg/ml and 41 x $10^{-12}$ at a fibrinogen concentration of 12.5 mg/ml. This experiment demonstrates that the fibrin material utilizing PBS as a buffer is less permeable than classic fibrin materials, thus capable of retaining more water. The diameters of fibers are also affected by the buffer selected. For a thrombin concentration of 250 IU/ml diluted with NaCl at 0.15 M, the fibers have a diameter of 0.073 $\mu$m at a fibrinogen concentration of 25 mg/ml and 0.083 $\mu$m at a fibrinogen concentration of 12.5 mg/ml. Using PBS as a buffer yields fibers with a diameter of 0.057 $\mu$m at a fibrinogen concentration of 25 mg/ml and 0.077 $\mu$m at a fibrinogen concentration of 12.5 mg/ml. Additionally, the mass length ratio is also affected by the buffer selected to reconstitute the fibrinogen and thrombin. At a thrombin concentration of 250 IU/ml buffered with NaCl at 0.15 M, the mass length ratio is 3.78 x $10^{12}$ at a fibrinogen concentration of 25 mg/ml and 4.83 x $10^{12}$ at a fibrinogen

concentration of 12.5 mg/ml. The use ofPBS as a buffer yields fibers with amass length ratio of 2.36 x $10^{12}$ at a fibrinogen concentration of 25 mg/ml and 4.24 x $10^{12}$ at a fibrinogen concentration of 12.5 mg/ml.

These experiments illustrate that the type ofbuffer used, differently effects the permeability factor as shown for NaCl and PBS (Tables 7 and 8). These experiments demonstrate that the concentration of thrombin has no effect on the permeability factor, fiber diameter, and mass length ratio as well when PBS, and not NaCl, is the buffer. PBS is an admixture composed of 0.13 M NaCl (800 mg/ L), KCL (20 mg/L), anhydrous $Na_2HPO_4$ (115 mg/L), and $KH_2PO_4$. Thus, PBS buffer contains NaCl at a molarity of nearly the NaCl 0.015 M buffer described in Tables 7 and 8. As the data shows, phosphate is therefore the complexing agent of the endogenous calcium. Tables summarizing these experiments are labeled Table 7 and Table 8 below.

Table 7 - Thrombin 2 IU/ml

| Buffer | Fibrinogen Concentration | Permeability ($K_S$) | Fiber Diameter $\mu$m | mass length ratio |
|---|---|---|---|---|
| NaCl 0.15 M | 25 mg/ml | 30.6 x $10^{-12}$ | 0.107 | 8.1 x $10^{12}$ |
| PBS | 25 mg/ml | 6.9 x $10^{-12}$ | 0.051 | 1.83 x $10^{-12}$ |
| NaCl 0.15 M | 12.5 mg/ml | 136 x $10^{-12}$ | 0.14 | 13.9 x $10^{12}$ |
| PBS | 12.5 mg/ml | 39.5 x $10^{-12}$ | 0.075 | 4.03 x $10^{12}$ |

Table 8 - Thrombin 250 IU/ml

| Buffer | Fibrinogen Concentration | Permeability (Ks) | Fiber Diameter $\mu$m | mass length ratio |
|---|---|---|---|---|
| NaCl 0.15 M | 25 mg/ml | 14.24 x $10^{-12}$ | 0.073 | 3.78 x $10^{12}$ |
| PBS | 25 mg/ml | 8.9 x $10^{-12}$ | 0.057 | 2.36 x $10^{12}$ |
| NaCl 0.15 M | 12.5 mg/ml | 47.7 x $10^{-12}$ | 0.083 | 4.83 x $10^{12}$ |
| PBS | 12.5 mg/ml | 41 x $10^{-12}$ | 0.077 | 4.24 x $10^{12}$ |

Calculation for permeability (Ks):

$$\frac{\text{Flow (ml/sec) x time to clot x viscosity } (10^2)}{\text{Pressure x Surface area of clot}}$$

Calculation for fiber diameter:

$$D^2 = 44.1 \text{ x Ks x concentration of fibrinogen } (X^{1.3736})$$

Calculation for mass length ratio:

$$\mu = \prod \text{ x } D^2 \text{ x } C/4X, \text{ where } C = 4.36 \text{ g/cm}^3$$

where C = 4.36 g/$cm^3$

The role of PBS as a complexing agent can also be seen by gel electrophoresis studies of fibrin materials. When an electric current is applied to an SDS-polyacrylimide-gelelectrophoresis containing fibrin hydrogel material prepared with PBS, little or no gamma-gamma banding can be seen while a fibronectin band may be viewed. This demonstrates that the PBS complexes calcium so that calcium is not available to collaterally associate fibrin through cross-linking. Classic fibrin materials show distinctive, strong gamma-gamma band, and no fibronectin band, when prepared with water or NaCl as a buffer. NaCl buffer with a molarity of 0.15 cannot block the action of calcium. The NaCl buffer's inability to block the action of calcium allows calcium to play its traditional role in collaterally associating fibrin, thus allowing thrombin to affect the pore size of the fibrin material. By acting as a complexing agent for endogenous calcium, PBS substantially removes thrombin's ability to affect pore size. Figure 9 below illustrates the classic fibrin material in gel electrophoresis. Figure 10 below illustrates the fibrin hydrogel material in gel electrophoresis.

The fibrin hydrogel materials have also been determined to contain anti-adhesive properties. Tables 9 and 10 below illustrate the anti-adhesive properties of the fibrin hydrogel materials. Table 9 shows the results of a side model study on rats. The caecum and interfacing parietal wall were abraded sufficiently to cause bleeding. The bleeding surfaces were cauterized to stop the bleeding on the injured surfaces in both control and test animals. Two types of fibrin hydrogel materials were formed between the injured surface. Fibrin film 1 (FF1) differs from fibrin film 2 (FF2) in that FF2 was compressed to release some water. The results show that the wounds treated with either normal fibrin hydrogel material (FF1) or compressed fibrin hydrogel material (FF2) have no adhesions between the caecum surface and the parietal surface. The control group for Table 9 had no fibrin hydrogel material applied, resulting in level 3 (the most severe) adhesions between the caecum surface and the parietal surface.

Table 10 shows the anti-adhesion properties of hydrogel fibrin glue material. This hydrogel material polymerizes within the body of the animal upon application using a delivery device such as that shown in Figure 6. The fibrin hydrogel glue was applied directly to the wound on the caecum surface, as well as to the parietal surface. All animals that received this fibrin hydrogel glue treatment were free of adhesion. In a second trial, a pre-cast fibrin hydrogel material was positioned between injured surfaces. Using a pre-cast fibrin hydrogel material demonstrated significant anti-adhesion properties as well.

Table 9

| Animal | Product Applied | Result |
|---|---|---|
| Control Rat 1 | none | adhesion (level 3) |
| Control Rat 2 | none | adhesion (level 3) |
| Control Rat 3 | none | adhesion (level 3) |
| Control Rat 4 | none | adhesion (level 3) |
| Rat 1 | FF1 | no adhesion |
| Rat 2 | FF1 | no adhesion |
| Rat 3 | FF2 | no adhesion |
| Rat 4 | FF2 | no adhesion |

Table 10

| Group Type | Number of Individuals | Thrombin Concentration | Result |
|---|---|---|---|
| Control | 5 | - | adhesion (level 3) |
| Pre-cast Hydrogel Fibrin Film | 5 | 100 IU/ml | 20 % adhesion |
| Hydrogel Fibrin Glue | 5 | 100 IU/ml | 0 % adhesion |

## Claims

**1.** A fibrin hydrogel material comprising:

a fibrin material formed in the absence of a calcium containing solution or in the presence of a solution which is capable of scavenging calcium ions associated with fibrinogen molecules, which fibrin material has a water content of at least 90% by weight of the material and retains at least about 80% of the water upon compression by centrifugation at 625G for 30 minutes, and is substantially free of cross-linking.

**2.** The hydrogel of Claim 1 wherein the hydrogel has a pore size within the range of from about 0.1 $\mu$m to about 5.0 $\mu$m.

**3.** The material of Claim 1 wherein the material is produced using an essentially calcium free thrombin solution.

**4.** The material of Claim 1 having an optical clarity of less than about 1.0 AUFS when measured with a spectrophotometer at 800 nm.

5. The material of Claim 1 substantially free of gamma-gamma cross-linking, as determined through gel electrophoresis.

6. The material of Claim 1 having anti-adhesive properties.

7. A multiple layer fibrin material comprising:

a fibrin glue layer; and
a layer of the fibrin hydrogel material of Claim 1.

8. A multiple layer fibrin material comprising:

a fibrin glue layer; and
a layer of the fibrin hydrogel material of Claim 1, which is a therapeutic fibrin hydrogel layer.

9. The multiple layer of fibrin material of Claim 8 further comprising:

a fibrin film layer; wherein the fibrin glue layer attaches the fibrin film layer to the therapeutic fibrin hydrogel layer.

10. A multiple layer fibrin material comprising:

a fibrin film layer; and
a layer of the fibrin hydrogel material of Claim 1.

11. The multiple layer fibrin material of Claim 7 or 8 wherein the fibrin glue layer has a pore size from about 2 μm to about 10 μm.

12. The multiple layer fibrin material of Claim 7 further including a fibrin layer, and being substantially cross-linked.

13. The multiple layer fibrin material of Claim 7 or 10 wherein the fibrin hydrogel layer has a pore size from about 0.1 μm to about 5 μm;

14. The multiple layer fibrin material of any one of Claims 7, 9 or 10 wherein the multiple layer fibrin material has anti-adhesive properties.

15. The multiple layer fibrin material of any one of Claims 7, 8 or 9 wherein the hydrogel layer releasably retains a therapeutic agent.

16. The multiple layer fibrin material of Claim 10 further including a therapeutic fibrin hydrogel layer.

17. The multiple layer fibrin material of Claim 16 wherein the therapeutic hydrogel layer releasably retains a therapeutic agent.

18. The multiple layer fibrin material of any one of Claims 7, 8, 9 or 17 wherein the therapeutic agent is selected from the group consisting of antibiotics, fibrinolytic agents and biological response modifiers.

19. The multiple layer fibrin material of any one of Claims 7, 8, 9 or 17 wherein the therapeutic agent comprises a pharmaceutical compound.

20. The multiple layer fibrin material of any one of Claims 7, 8, 9 or 17 wherein the therapeutic agent comprises living cells.

21. The multiple layer fibrin material of any one of Claims 8, 9 or 16 wherein the therapeutic fibrin hydrogel layer has a pore size from about 0.1 μm to about 5 μm.

22. The multiple layer fibrin material of Claim 9 or 10 wherein the fibrin film layer has a pore size from about 2 μm to about 10 μm.

23. The fibrin hydrogel material of any of Claims 1 to 6 or the multiple layer fibrin material of any one of Claims 7 to 22 for use in medicine.

**24.** Use of a first fibrin material in the manufacture of a medicament for treating a patient to be treated with a second fibrin material, wherein the first fibrin material has a pore size from 2 μm to 10 μm; and the second fibrin material has a pore size of from 0.1 μm to 5 μm and is capable of retaining 80-90% of water upon compression by centrifugation at 625G for 30 minutes, and is substantially free of cross-linking, and was formed in the absence of a calcium containing solution or in the presence of a solution which is capable of scavenging calcium ions associated with fibrinogen molecules; and wherein the first fibrin material is for applying to a surface and the second fibrin material is to be applied to the first fibrin material.

**25.** Use of a second fibrin material in the manufacture of a medicament for treating a patient to be treated with a first fibrin material, wherein the first fibrin material has a pore size from 2 μm to 10 μm; and the second fibrin material has a pore size of from 0.1 μm to 5 μm and is capable of retaining 80-90% of water upon compression by centrifugation at 625G for 30 minutes, and is substantially free of cross-linking, and was formed in the absence of a calcium containing solution or in the presence of a solution which is capable of scavenging calcium ions associated with fibrinogen molecules; and wherein the first fibrin material is to be applied to a surface and the second fibrin material is for applying to the first fibrin material.

**26.** A first fibrin material for use in treating a patient to be treated with a second fibrin material, wherein the first fibrin material has a pore size from 2 μm to 10 μm; and the second fibrin material has a pore size of from 0.1 μm to 5 μm and is capable of retaining 80-90% of water upon compression by centrifugation at 625G for 30 minutes, and is substantially free of cross-linking, and was formed in the absence of a calcium containing solution or in the presence of a solution which is capable of scavenging calcium ions associated with fibrinogen molecules; and wherein the first fibrin material is for use by applying to a surface and the second fibrin material is to be applied to the first fibrin material.

**27.** A second fibrin material for use in treating a patient to be treated with a first fibrin material, wherein the first fibrin material has a pore size from 2 μm to 10 μm; and the second fibrin material has a pore size of from 0.1 μm to 5 μm and is capable of retaining 80-90% of water upon compression by centrifugation at 625G for 30 minutes, and is substantially free of cross-linking, and was formed in the absence of a calcium containing solution or in the presence of a solution which is capable of scavenging calcium ions associated with fibrinogen molecules; and wherein the first fibrin material is to be applied to a surface and the second fibrin material is for use by applying to the first fibrin material.

**28.** The use of Claim 24 or 25 or the fibrin material of Claim 26 or 27 wherein the first fibrin material is provided by:

providing thrombin solution having a concentration from about 50 IU/ml to about 300 IU/ml;
providing fibrinogen solution having a concentration from about 1.5 mg/ml to about 100 mg/ml; and
mixing the thrombin solution with the fibrinogen solution.

**29.** The use of Claim 24 or 25 or the fibrin material of Claim 26 or 27 wherein the second fibrin material is provided by:

providing thrombin solution having a concentration from about 1 IU/ml to about 300 IU/ml;
providing fibrinogen solution having a concentration from about 1.5 mg/ml to about 100 mg/ml;
providing a chelating agent as an antagonist to fibrinopeptide transamidation reactions; and
mixing the thrombin solution, the fibrinogen solution, and the chelating agent.

**30.** The use or fibrin material of Claim 29 wherein the diluent is phosphate buffered saline.

**Patentansprüche**

**1.** Fibrinhydrogelmaterial, umfassend:

ein Fibrinmaterial, ausgebildet in Abwesenheit einer calciumhaltigen Lösung oder in Gegenwart einer Lösung, die in der Lage ist, mit Fibrinogenmolekülen assoziierte Calciumionen abzufangen, wobei das Fibrinmaterial einen Wassergehalt von mindestens 90 Gew.-%, bezogen auf das Material, aufweist und mindestens etwa 80 % des Wassers nach Kompression durch Zentrifugierung mit 625G für 30 Minuten zurückbehält und im Wesentlichen frei von Vernetzung ist.

2. Hydrogel gemäß Anspruch 1, wobei das Hydrogel eine Porengröße im Bereich von etwa 0,1 µm bis etwa 5,0 µm aufweist.

3. Material gemäß Anspruch 1, wobei das Material unter Verwendung einer im Wesentlichen calciumfreien Thrombinlösung hergestellt wird.

4. Material gemäß Anspruch 1, das eine optische Klarheit von weniger als etwa 1,0 AUFS aufweist, gemessen mit einem Spektrophotometer bei 800 nm.

5. Material gemäß Anspruch 1, das im Wesentlichen frei von Gamma-Gamma-Vernetzung ist, bestimmt durch Gelelektrophorese.

6. Material gemäß Anspruch 1, das antiadhäsive Eigenschaften aufweist.

7. Mehrschichtiges Fibrinmaterial, umfassend:

   eine Fibrinkleberschicht; und
   eine Schicht aus dem Fibrinhydrogelmaterial gemäß Anspruch 1.

8. Mehrschichtiges Fibrinmaterial, umfassend:

   eine Fibrinkleberschicht; und
   eine Schicht aus dem Fibrinhydrogelmaterial gemäß Anspruch 1, die eine therapeutische Fibrinhydrogelschicht ist.

9. Mehrschichtiges Fibrinmaterial gemäß Anspruch 8, ferner umfassend:

   eine Fibrinfilmschicht, wobei die Fibrinkleberschicht die Fibrinfilmschicht an der therapeutischen Fibrinhydrogelschicht befestigt.

10. Mehrschichtiges Fibrinmaterial, umfassend:

   eine Fibrinfilmschicht; und
   eine Schicht aus dem Fibrinhydrogelmaterial gemäß Anspruch 1.

11. Mehrschichtiges Fibrinmaterial gemäß Anspruch 7 oder 8, wobei die Fibrinkleberschicht eine Porengröße von etwa 2 µm bis etwa 10 µm aufweist.

12. Mehrschichtiges Fibrinmaterial gemäß Anspruch 7, das ferner eine Fibrinschicht umfasst und im Wesentlichen vernetzt ist.

13. Mehrschichtiges Fibrinmaterial gemäß Anspruch 7 oder 10, wobei die Fibrinhydrogelschicht eine Porengröße von etwa 0,1 µm bis etwa 5 µm aufweist.

14. Mehrfache Schicht von Fibrinmaterial gemäß einem der Ansprüche 7, 9 oder 10, wobei das mehrschichtige Fibrinmaterial antiadhäsive Eigenschaften aufweist.

15. Mehrschichtiges Fibrinmaterial gemäß einem der Ansprüche 7, 8 oder 9, wobei die Hydrogelschicht ein Therapeutikum freisetzbar zurückbehält.

16. Mehrschichtiges Fibrinmaterial gemäß Anspruch 10, das ferner eine therapeutische Fibrinhydrogelschicht umfasst.

17. Mehrschichtiges Fibrinmaterial gemäß Anspruch 16, wobei die therapeutische Hydrogelschicht einen Wirkstoff freisetzbar zurückbehält.

18. Mehrschichtiges Fibrinmaterial gemäß einem der Ansprüche 7, 8, 9 oder 17, wobei der Wirkstoff ausgewählt ist aus der Gruppe bestehend aus Antibiotika, Fibrinolytika und Modifikatoren der biologischen Reaktion.

**19.** Mehrschichtiges Fibrinmaterial gemäß einem der Ansprüche 7, 8, 9 oder 17, wobei das Therapeutikum eine pharmazeutische Verbindung umfasst.

**20.** Mehrschichtiges Fibrinmaterial gemäß einem der Ansprüche 7, 8, 9 oder 17, wobei das Therapeutikum lebende Zellen umfasst.

**21.** Mehrschichtiges Fibrinmaterial gemäß einem der Ansprüche 8, 9 oder 16, wobei die therapeutische Fibrinhydrogelschicht eine Porengröße von etwa 0,1 μm bis etwa 5 μm aufweist.

**22.** Mehrschichtiges Fibrinmaterial gemäß Anspruch 9 oder 10, wobei die Fibrinfilmschicht eine Porengröße von etwa 2 μm bis etwa 10 μm aufweist.

**23.** Fibrinhydrogelmaterial gemäß einem der Ansprüche 1 bis 6 oder mehrschichtiges Fibrinmaterial gemäß einem der Ansprüche 7 bis 22 zur Verwendung in der Medizin.

**24.** Verwendung eines ersten Fibrinmaterials bei der Herstellung eines Medikaments zum Behandeln eines mit einem zweiten Fibrinmaterial zu behandelnden Patienten, wobei das erste Fibrinmaterial eine Porengröße von 2 μm bis 10 μm aufweist; und das zweite Fibrinmaterial eine Porengröße von 0,1 μm bis 5 μm aufweist und in der Lage ist, 80-90 % Wasser nach Kompression durch Zentrifugierung mit 625G für 30 Minuten zurückzubehalten, und im Wesentlichen frei von Vernetzung ist und in Abwesenheit einer calciumhaltigen Lösung oder in Gegenwart einer Lösung, die in der Lage ist, mit Fibrinogenmolekülen assoziierte Calciumionen abzufangen, ausgebildet wurde; und wobei das erste Fibrinmaterial zum Aufbringen auf eine Oberfläche vorgesehen ist und das zweite Fibrinmaterial auf das erste Fibrinmaterial aufzubringen ist.

**25.** Verwendung eines zweiten Fibrinmaterials bei der Herstellung eines Medikaments zum Behandeln eines mit einem ersten Fibrinmaterial zu behandelnden Patienten, wobei das erste Fibrinmaterial eine Porengröße von 2 μm bis 10 μm aufweist; und das zweite Fibrinmaterial eine Porengröße von 0,1 μm bis 5 μm aufweist und in der Lage ist, 80-90 % Wasser nach Kompression durch Zentrifugierung mit 625G für 30 Minuten zurückzubehalten, und im Wesentlichen frei von Vernetzung ist und in Abwesenheit einer calciumhaltigen Lösung oder in Gegenwart einer Lösung, die in der Lage ist, mit Fibrinogenmolekülen assoziierte Calciumionen abzufangen, ausgebildet wurde; und wobei das erste Fibrinmaterial auf eine Oberfläche aufzubringen ist und das zweite Fibrinmaterial zum Aufbringen auf das erste Fibrinmaterial vorgesehen ist.

**26.** Erstes Fibrinmaterial zur Verwendung beim Behandeln eines mit einem zweiten Fibrinmaterial zu behandelnden Patienten, wobei das erste Fibrinmaterial eine Porengröße von 2 μm bis 10 μm aufweist; und das zweite Fibrinmaterial eine Porengröße von 0,1 μm bis 5 μm aufweist und in der Lage ist, 80-90 % Wasser nach Kompression durch Zentrifugierung mit 625G für 30 Minuten zurückzubehalten, und im Wesentlichen frei von Vernetzung ist und in Abwesenheit einer calciumhaltigen Lösung oder in Gegenwart einer Lösung, die in der Lage ist, mit Fibrinogenmolekülen assoziierte Calciumionen abzufangen, ausgebildet wurde; und wobei das erste Fibrinmaterial zur Verwendung durch Aufbringen auf eine Oberfläche vorgesehen ist und das zweite Fibrinmaterial auf das erste Fibrinmaterial aufzubringen ist.

**27.** Zweites Fibrinmaterial zur Verwendung beim Behandeln eines mit einem ersten Fibrinmaterial zu behandelnden Patienten, wobei das erste Fibrinmaterial eine Porengröße von 2 μm bis 10 μm aufweist; und das zweite Fibrinmaterial eine Porengröße von 0,1 μm bis 5 μm aufweist und in der Lage ist, 80-90 % Wasser nach Kompression durch Zentrifugierung mit 625G für 30 Minuten zurückzubehalten, und im Wesentlichen frei von Vernetzung ist und in Abwesenheit einer calciumhaltigen Lösung oder in Gegenwart einer Lösung, die in der Lage ist, mit Fibrinogenmolekülen assoziierte Calciumionen abzufangen, ausgebildet wurde; und wobei das erste Fibrinmaterial auf eine Oberfläche aufzubringen ist und das zweite Fibrinmaterial zur Verwendung durch Aufbringen auf das erste Fibrinmaterial vorgesehen ist.

**28.** Verwendung gemäß Anspruch 24 oder 25 oder Fibrinmaterial gemäß Anspruch 26 oder 27, wobei das erste Fibrinmaterial bereitgestellt wird durch:

Bereitstellen einer Thrombinlösung mit einer Konzentration von etwa 50 IU/ml bis etwa 300 IU/ml;
Bereitstellen einer Fibrinogenlösung mit einer Konzentration von etwa 1,5 mg/ml bis etwa 100 mg/ml; und
Mischen der Thrombinlösung mit der Fibrinogenlösung.

**29.** Verwendung gemäß Anspruch 24 oder 25 oder Fibrinmaterial gemäß Anspruch 26 oder 27, wobei das zweite Fibrinmaterial bereitgestellt wird durch:

Bereitstellen einer Thrombinlösung mit einer Konzentration von etwa 1 IU/ml bis etwa 300 IU/ml;
Bereitstellen einer Fibrinogenlösung mit einer Konzentration von etwa 1,5 mg/ml bis etwa 100 mg/ml;
Bereitstellen eines Chelators als Antagonist für Fibrinopeptidtransamidierungsreaktionen; und
Mischen der Thrombinlösung, der Fibrinogenlösung und des Chelators.

**30.** Verwendung oder Fibrinmaterial gemäß Anspruch 29, wobei das Verdünnungsmittel eine Phosphat-gepufferte Salzlösung ist.

**Revendications**

**1.** Matière à base d'hydrogel de fibrine comprenant :

une matière à base de fibrine formée en l'absence d'une solution contenant du calcium ou en présence d'une solution qui est capable d'éliminer des ions calcium associés à des molécules de fibrinogène, laquelle matière à base de fibrine a une teneur en eau d'au moins 90 % en poids de la matière et retient au moins environ 80 % de l'eau après compression par centrifugation à 625 g pendant 30 minutes, et est sensiblement exempte de réticulation.

**2.** Hydrogel selon la revendication 1, dans lequel l'hydrogel a une grosseur de pores dans la plage d'environ 0,1 μm à environ 5,0 μm.

**3.** Matière selon la revendication 1, dans laquelle la matière est produite en utilisant une solution de thrombine sensiblement exempte de calcium.

**4.** Matière selon la revendication 1 ayant une clarté optique inférieure à environ 1,0 AUFS lorsqu'elle est mesurée avec un spectrophotomètre à 800 nm.

**5.** Matière selon la revendication 1 sensiblement exempte de réticulation gamma-gamma, telle qu'elle est déterminée par électrophorèse sur gel.

**6.** Matière selon la revendication 1 ayant des propriétés antiadhésives.

**7.** Matière à base de fibrine multicouche comprenant :

une couche de colle de fibrine ; et
une couche de la matière à base d'hydrogel de fibrine selon la revendication 1.

**8.** Matière à base de fibrine multicouche comprenant :

une couche de colle de fibrine ; et
une couche de la matière à base d'hydrogel de fibrine selon la revendication 1, qui est une couche d'hydrogel de fibrine thérapeutique.

**9.** Multicouche de la matière à base de fibrine selon la revendication 8 comprenant en outre :

une couche de film de fibrine ; dans laquelle la couche de colle de fibrine fixe la couche de film de fibrine à la couche d'hydrogel de fibrine thérapeutique.

**10.** Matière à base de fibrine multicouche comprenant :

une couche de film de fibrine ; et
une couche de la matière à base d'hydrogel de fibrine selon la revendication 1.

**11.** Matière à base de fibrine multicouche selon la revendication 7 ou 8, dans laquelle la couche de colle de fibrine a une grosseur de pores d'environ 2 μm à environ 10 μm.

**12.** Matière à base de fibrine multicouche selon la revendication 7 comprenant en outre une couche de fibrine, et sensiblement réticulée.

**13.** Matière à base de fibrine multicouche selon la revendication 7 ou 10, dans laquelle la couche d'hydrogel de fibrine a une grosseur de pores d'environ 0,1 μm à environ 5 μm.

**14.** Matière à base de fibrine multicouche selon l'une quelconque des revendications 7, 9 ou 10, dans laquelle la matière à base de fibrine multicouche a des propriétés antiadhésives.

**15.** Matière à base de fibrine multicouche selon l'une quelconque des revendications 7, 8 ou 9, dans laquelle la couche d'hydrogel retient de manière à le libérer un agent thérapeutique.

**16.** Matière à base de fibrine multicouche selon la revendication 10 comprenant en outre une couche d'hydrogel de fibrine thérapeutique.

**17.** Matière à base de fibrine multicouche selon la revendication 16, dans laquelle la couche d'hydrogel thérapeutique retient de manière à la libérer un agent thérapeutique.

**18.** Matière à base de fibrine multicouche selon l'une quelconque des revendications 7,8, 9 ou 17, dans laquelle l'agent thérapeutique est sélectionné dans le groupe constitué par les antibiotiques, les agents fibrinolytiques et les modificateurs de réponse biologique.

**19.** Matière à base de fibrine multicouche selon l'une quelconque des revendications 7, 8, 9 ou 17, dans laquelle l'agent thérapeutique comprend un composé pharmaceutique.

**20.** Matière à base de fibrine multicouche selon l'une quelconque des revendications 7, 8, 9 ou 17, dans laquelle l'agent thérapeutique comprend des cellules vivantes.

**21.** Matière à base de fibrine multicouche selon l'une quelconque des revendications 8, 9 ou 16, dans laquelle la couche d'hydrogel de fibrine thérapeutique a une grosseur de pores d'environ 0,1 μm à environ 5 μm.

**22.** Matière à base de fibrine multicouche selon la revendication 9 ou 10, dans laquelle la couche de film de fibrine a une grosseur de pores d'environ 2 μm à environ 10 μm.

**23.** Matière à base d'hydrogel de fibrine selon l'une quelconque des revendications 1 à 6 ou matière à base de fibrine multicouche selon l'une quelconque des revendications 7 à 22 destinée à une utilisation en médecine.

**24.** Utilisation d'une première matière à base de fibrine dans la fabrication d'un médicament pour traiter un patient à traiter avec une deuxième matière à base de fibrine, dans laquelle la première matière à base de fibrine a une grosseur de pores de 2 μm à 10 μm ; et la deuxième matière à base de fibrine a une grosseur de pores de 0,1 μm à 5 μm et est capable de retenir 80-90 % de l'eau après compression par centrifugation à 625 g pendant 30 minutes, et est sensiblement exempte de réticulation, et a été formée en l'absence d'une solution contenant du calcium ou en présence d'une solution qui est capable d'éliminer des ions calcium associés à des molécules de fibrinogène ; et dans laquelle la première matière à base de fibrine est destinée à être appliquée sur une surface et la deuxième matière à base de fibrine est à appliquer sur la première matière à base de fibrine.

**25.** Utilisation d'une deuxième matière à base de fibrine dans la fabrication d'un médicament pour traiter un patient à traiter avec une première matière à base de fibrine, dans laquelle la première matière à base de fibrine a une grosseur de pores de 2 μm à 10 μm ; et la deuxième matière à base de fibrine a une grosseur de pores de 0,1 μm à 5 μm et est capable de retenir 80-90 % de l'eau après compression par centrifugation à 625 g pendant 30 minutes, et est sensiblement exempte de réticulation, et a été formée en l'absence d'une solution contenant du calcium ou en présence d'une solution qui est capable d'éliminer des ions calcium associés à des molécules de fibrinogène ; et dans laquelle la première matière à base de fibrine est à appliquer sur une surface et la deuxième matière à base de fibrine est destinée à être appliquée sur la première matière à base de fibrine.

**26.** Première matière à base de fibrine destinée à une utilisation dans le traitement d'un patient à traiter avec une deuxième matière à base de fibrine, dans laquelle la première matière à base de fibrine a une grosseur de pores de 2 μm à 10 μm ; et la deuxième matière à base de fibrine a une grosseur de pores de 0,1 μm à 5 μm et est capable de retenir 80-90 % de l'eau après compression par centrifugation à 625 g pendant 30 minutes, et est sensiblement exempte de réticulation, et a été formée en l'absence d'une solution contenant du calcium ou en présence d'une solution qui est capable d'éliminer des ions calcium associés à des molécules de fibrinogène ; et dans laquelle la première matière à base de fibrine est destinée à une utilisation par application sur une surface et la deuxième matière à base de fibrine est à appliquer sur la première matière de fibrine.

**27.** Deuxième matière à base de fibrine destinée à une utilisation dans le traitement d'un patient à traiter avec une première matière à base de fibrine, dans laquelle la première matière à base de fibrine a une grosseur de pores de 2 μm à 10 μm ; et la deuxième matière à base de fibrine a une grosseur de pores de 0,1 μm à 5 μm et est capable de retenir 80-90 % de l'eau après compression par centrifugation à 625 g pendant 30 minutes, et est sensiblement exempte de réticulation, et a été formée en l'absence d'une solution contenant du calcium ou en présence d'une solution qui est capable d'éliminer des ions calcium associés à des molécules de fibrinogène ; et dans laquelle la première matière à base de fibrine est à appliquer sur une surface et la deuxième matière à base de fibrine est destinée à une utilisation par application sur la première matière à base de fibrine.

**28.** Utilisation selon la revendication 24 ou 25 ou matière à base de fibrine selon la revendication 26 ou 27, dans laquelle la première matière à base de fibrine est fournie :

en fournissant une solution de thrombine ayant une concentration d'environ 50 UI/ml à environ 300 UI/ml ;
en fournissant une solution de fibrinogène ayant une concentration d'environ 1,5 mg/ml à environ 100 mg/ml ; et
en mélangeant la solution de thrombine avec la solution de fibrinogène.

**29.** Utilisation selon la revendication 24 ou 25 ou matière à base de fibrine selon la revendication 26 ou 27, dans laquelle la deuxième matière à base de fibrine est fournie :

en fournissant une solution de thrombine ayant une concentration d'environ 1 UI/ml à environ 300 UI/ml ;
en fournissant une solution de fibrinogène ayant une concentration d'environ 1,5 mg/ml à environ 100 mg/ml ;
en fournissant un chélateur en tant qu'antagoniste des réactions de transamidation des fibrinopeptides ; et
en mélangeant la solution de thrombine, la solution de fibrinogène et le chélateur.

**30.** Utilisation ou matière à base de fibrine selon la revendication 29, dans laquelle le diluant est une solution saline tamponnée par les phosphates.

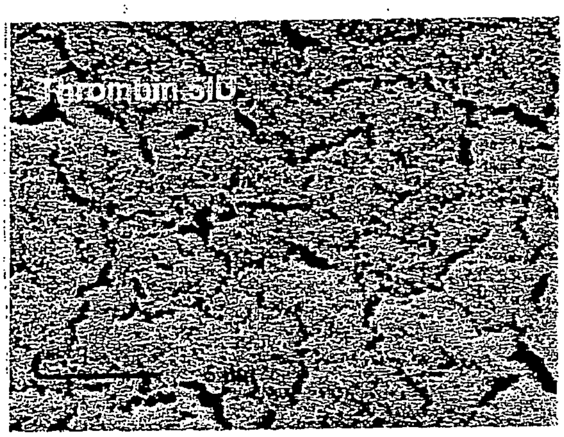


Fig. 1

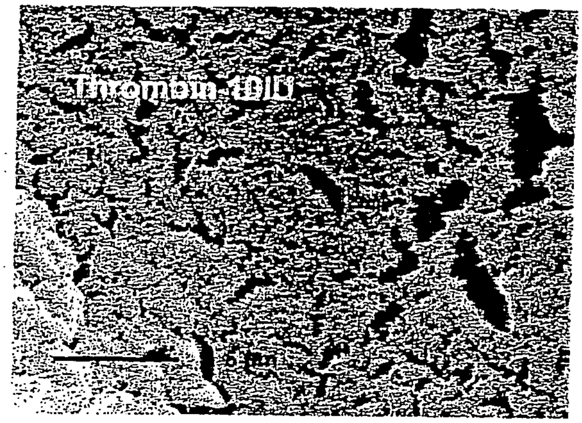


Fig. 2

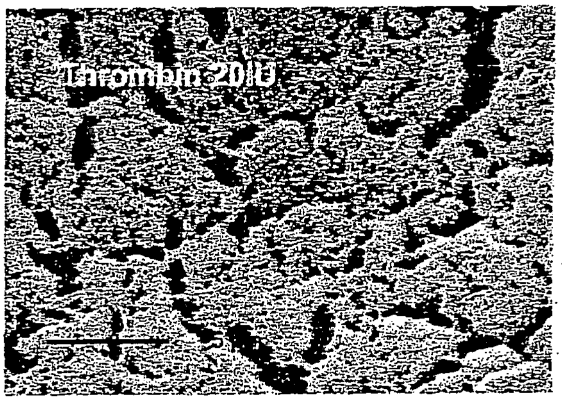


Fig. 3

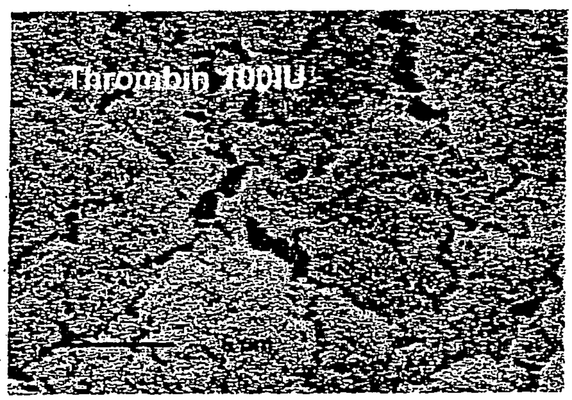


Fig. 4

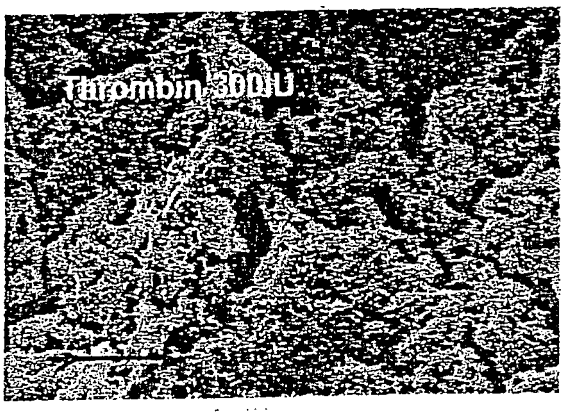


Fig. 5

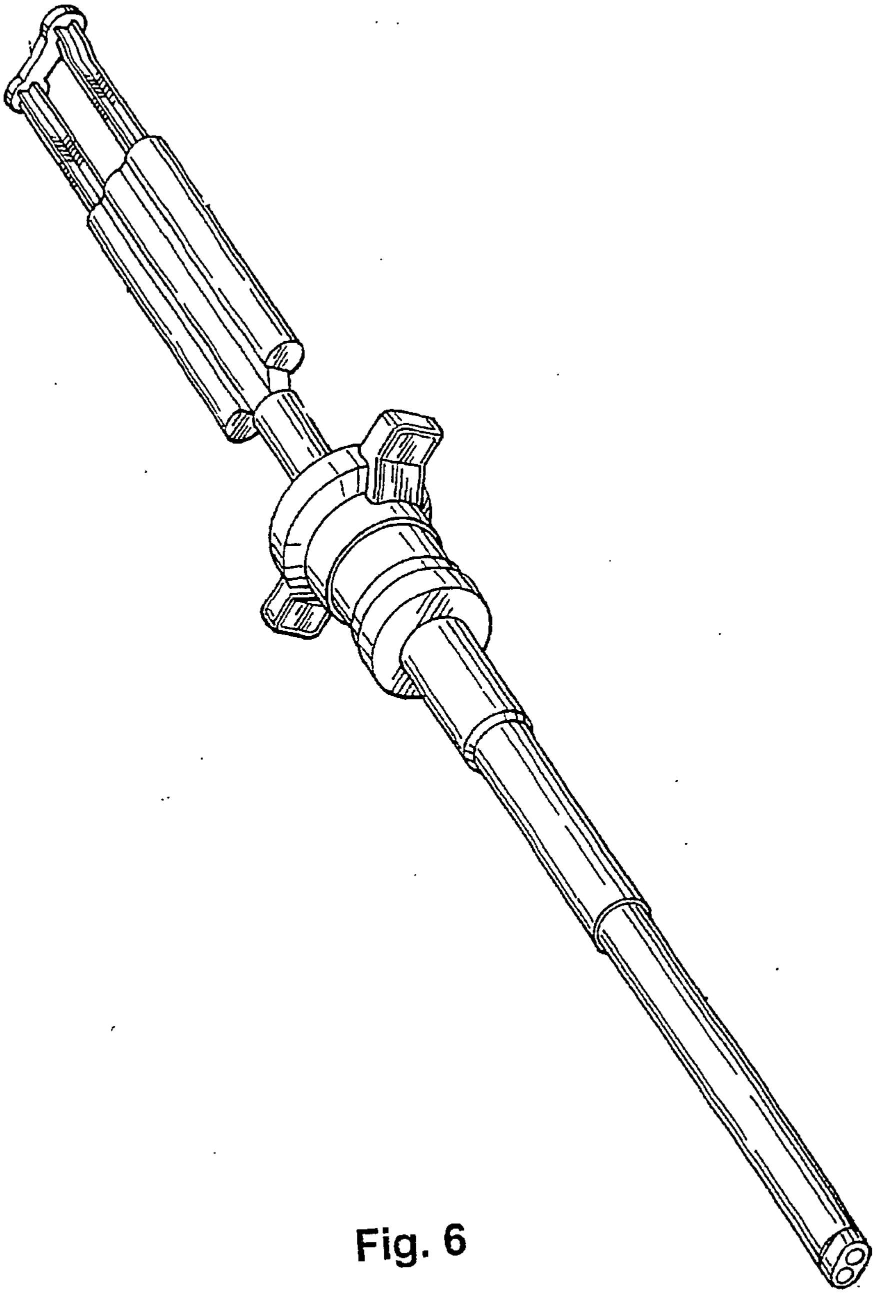

Fig. 6

Delivery device : pressurized can

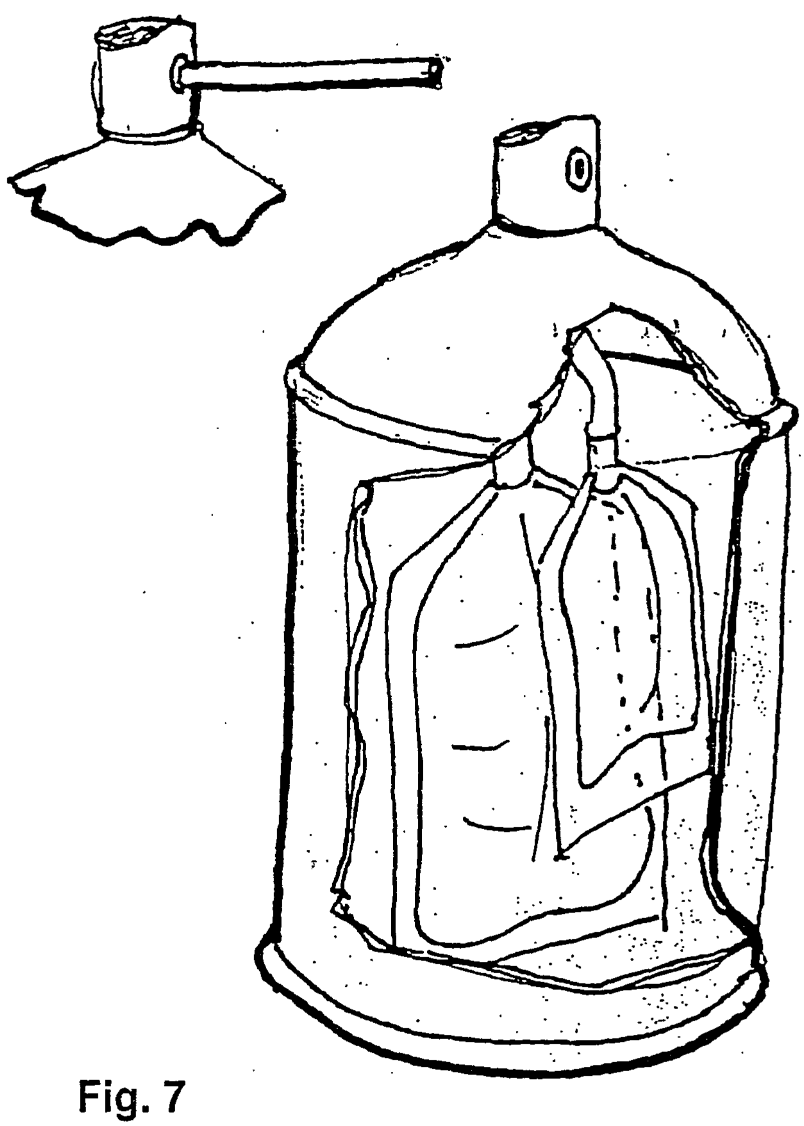

Fig. 7

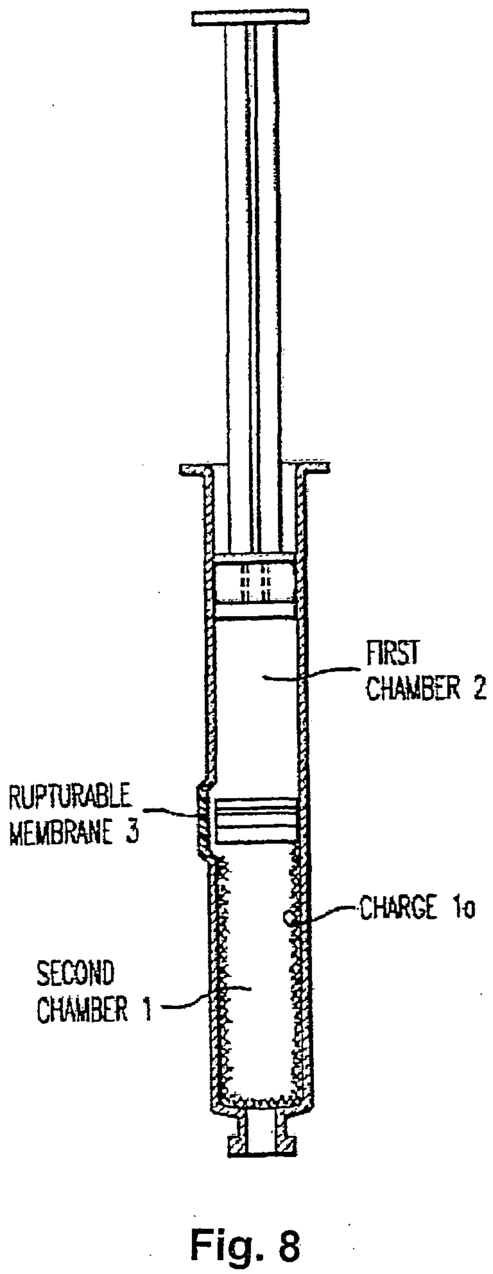
FIRST
CHAMBER 2
RUPTURABLE
MEMBRANE 3
CHARGE 1o
SECOND
CHAMBER 1

Fig. 8

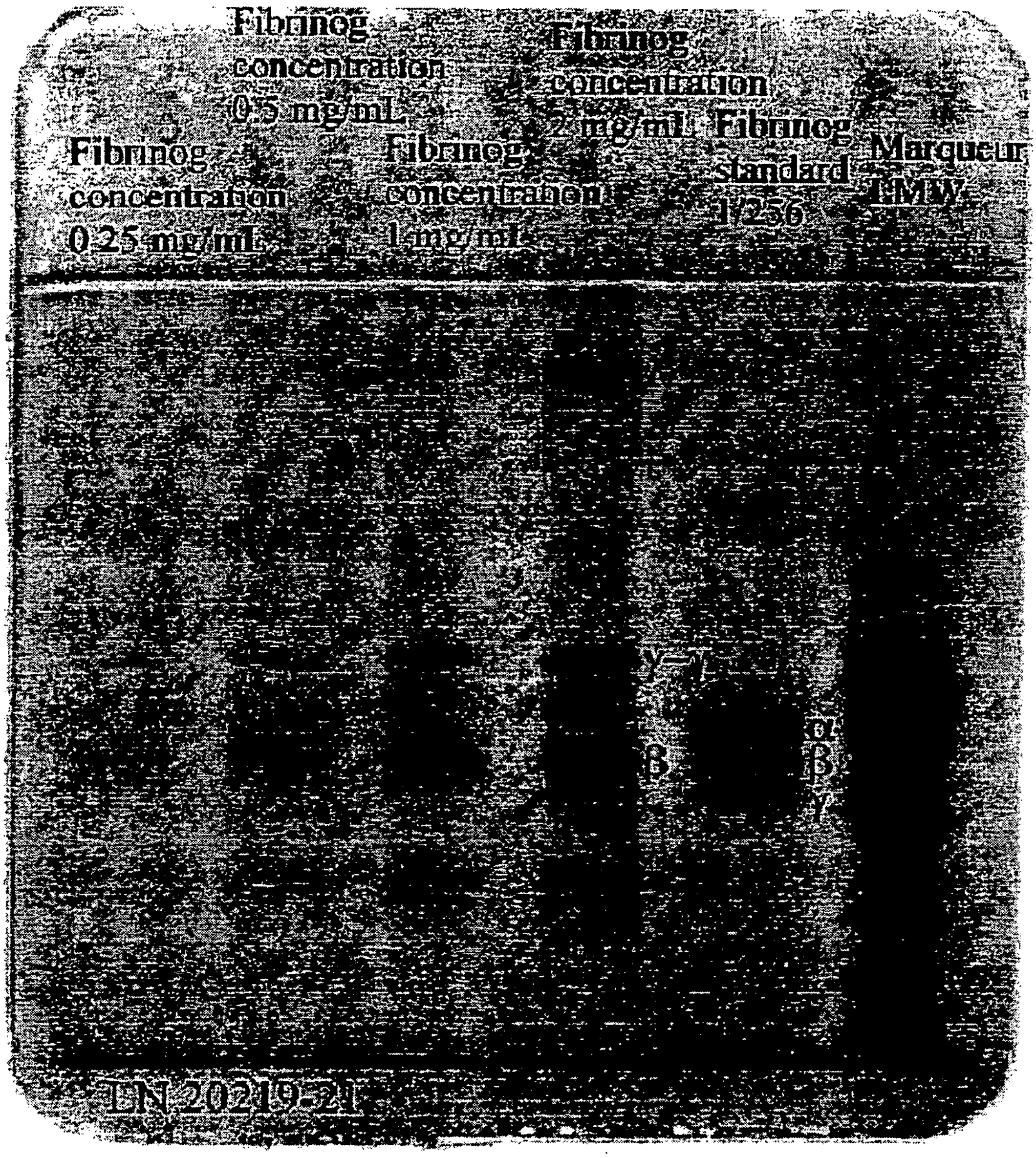
Fibrinog concentration 0.5 mg/mL
Fibrinog concentration 2 mg/mL
Fibrinog concentration 0.25 mg/mL
Fibrinog concentration 1 mg/mL
Fibrinog standard 1/256
Marqueur LMW
β
α
β
γ
LN 20219-21

FIG. 9

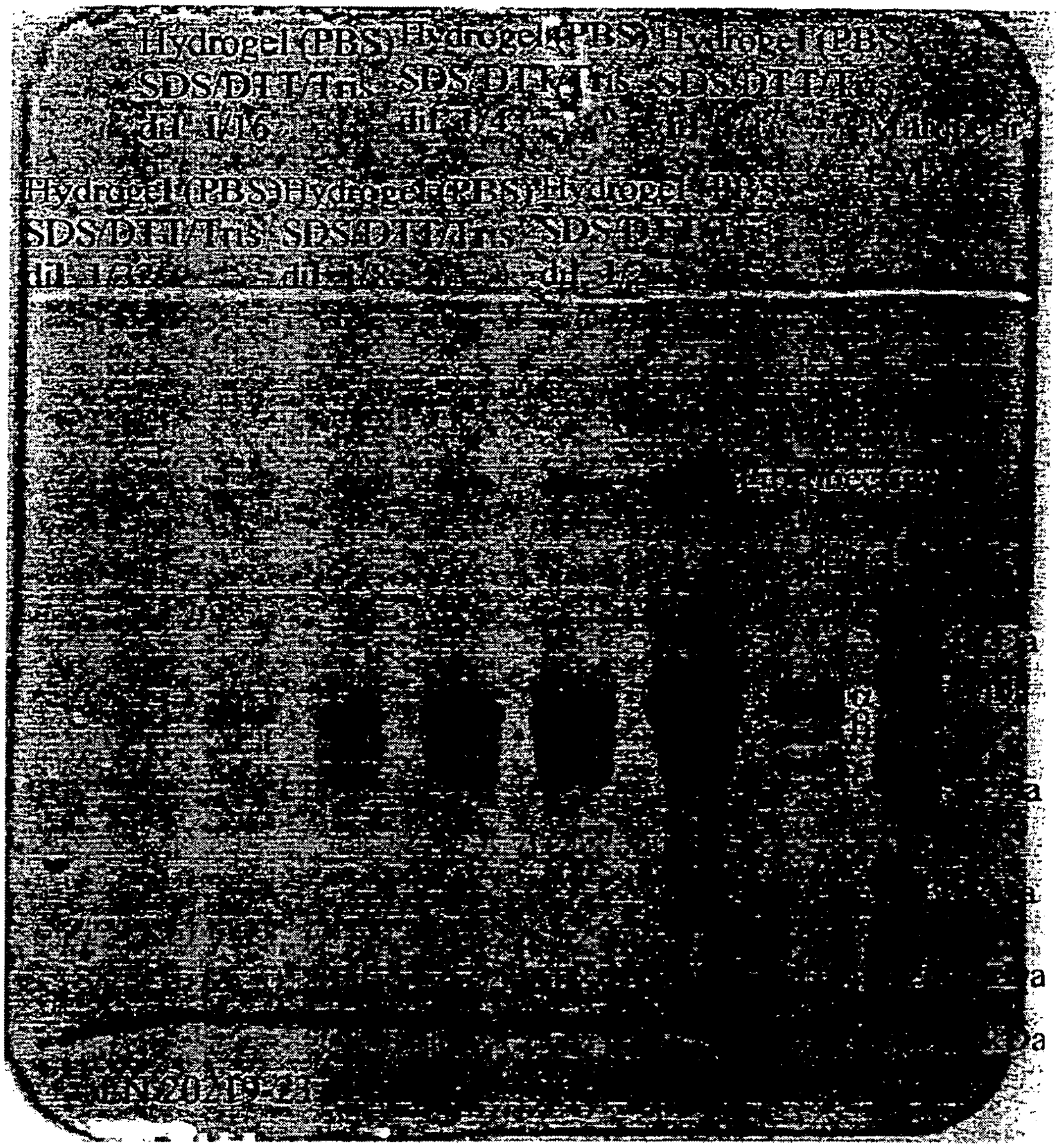
Hydrogel (PBS) Hydrogel (PBS) Hydrogel (PBS)
SDS/DTT/Tris SDS/DTT/Tris SDS/DTT/Tris
dil 1/16 dil 1/4
Hydrogel (PBS) Hydrogel (PBS) Hydrogel (PBS)
SDS/DTT/Tris SDS/DTT/Tris SDS/DTT/Tris
dil 1/8 dil 1/2

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9622115 A
- WO 9802098 A
- US 5989215 A


### Non-patent literature cited in the description


- **A.F. HANEY ; E. DOTY.** *Fertility and Sterility,* 1993, vol. 60, 550-558